(19) Europäisches Patentamt
European Patent Office
Office européen des brevets

(11) **EP 3 510 508 B1**

(12) **EUROPEAN PATENT SPECIFICATION**

(45) Date of publication and mention
of the grant of the patent:
**11.03.2026  Bulletin 2026/11**

(21) Application number: **17768202.8**

(22) Date of filing: **07.09.2017**

(51) International Patent Classification (IPC):
***G16H 50/20*** *(2018.01)*

(52) Cooperative Patent Classification (CPC):
**G16H 50/20**

(86) International application number:
**PCT/GB2017/052615**

(87) International publication number:
**WO 2018/046926 (15.03.2018 Gazette 2018/11)**

(54) **METHOD OF DISEASE CHARACTERISATION**

VERFAHREN ZUR KRANKHEITSCHARAKTERISIERUNG

PROCÉDÉ DE CARACTÉRISATION DE MALADIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.09.2016  GB 201615330**

(43) Date of publication of application:
**17.07.2019  Bulletin 2019/29**

(73) Proprietor: **The University of Birmingham
Birmingham, West Midlands B15 2TT (GB)**

(72) Inventors:
• **BUNTE, Kerstin
9746 RK Groningen (NL)**
• **ARLT, Wiebke
Birmingham
West Midlands B29 7EX (GB)**
• **TINO, Peter
Oldbury
West Midlands B68 9DE (GB)**

(74) Representative: **Barker Brettell LLP
100 Hagley Road
Edgbaston
Birmingham, West Midlands B16 8QQ (GB)**

(56) References cited:
**US-A1- 2012 040 332     US-A1- 2014 220 580**

• ANONYMOUS: "Learning vector quantization",
WIKIPEDIA, 6 August 2016 (2016-08-06), pages 1
- 3, XP055823531, Retrieved from the Internet
<URL:https://en.wikipedia.org/w/index.php?
title=Learning_vector_quantization&
oldid=733210216> [retrieved on 20210712]
• ANONYMOUS: "Cosine similarity - Wikipedia",
WIKIPEDIA, 30 January 2011 (2011-01-30),
XP055564550, Retrieved from the Internet
<URL:https://en.wikipedia.org/w/index.php?
title=Cosine_similarity&oldid=411040778>
[retrieved on 20190305]
• WIEBKE ARLT ET AL: "Urine Steroid
Metabolomics as a Biomarker Tool for Detecting
Malignancy in Adrenal Tumors", JOURNAL OF
CLINICAL ENDOCRINOLOGY & METABOLISM,
vol. 96, no. 12, 1 December 2011 (2011-12-01),
pages 3775 - 3784, XP055194028, ISSN:
0021-972X, DOI: 10.1210/jc.2011-1565

## Description

The invention relates to a method of characterising a disease state, identifying a disease state or following the progression of a disease state, utilising vectors with dimensions corresponding to different biomarkers. More particularly, the invention relates to a computer program for identifying a disease state involving collecting metabolic data from a plurality of subjects, the metabolic data including an amount of pregnenolone and an amount of metabolites of pregnenolone for each subject, and/or including an amount of 11-deoxycortisol and an amount of metabolites of 11-deoxycortisol for each subject, and presenting the data as vectors with dimensions corresponding to different biomarkers.

Due to improved biochemical sensor technology and biobanking in North America and Europe, the amounts of complex biomedical data are growing constantly. With the data also the demand for interpretable interdisciplinary analysis techniques increases. Further difficulties arise since biomedical data is often very heterogeneous, either due to the availability of measurements or individual differences in the biological processes. Urine steroid metabolomics is a novel biomarker tool for adrenal cortex function [1], WO 2010/092363, measured by gas chromatography-mass spectrometry (GC-MS), which is considered the reference standard for the biochemical diagnosis of inborn steroidogenic disorders. Steroidogenesis encompasses the complex process by which cholesterol is converted to biologically active steroid hormones. Inherited or inborn disorders of steroidogenesis result from genetic mutations which lead to defective production of any of the enzymes or a cofactor responsible for catalysing salt and glucose homeostasis, sex differentiation and sex specific development. Treatment involves replacing the deficient hormones which, if replaced adequately, will in turn suppress any compensatory up-regulation of other hormones that drive the disease process. Currently, up to 34 distinct steroid metabolite concentrations are extracted from a single GC-MS profile by automatic quantitation following selected-ion-monitoring (SIM) analysis, resulting in a 34 dimensional fingerprint vector. However, the interpretation of this fingerprint is difficult and requires enormous experience and expertise, which makes it a relatively inaccessible tool for most clinical endocrinologists.

Patent application publication US 2014/0220580 A1 relates to assessing biomarkers for diagnostic, therapy-related or prognostic methods to identify phenotypes, such as a condition or disease, or the stage or progression of a disease, select candidate treatment regimens for diseases, conditions, disease stages, and stages of a condition, and to determine treatment efficacy. Web page https://en.wikipedia.org/w/index.php?title=learning_vector_quantization&oldid=733210216 "Learning vector quantization" relates to learning vector quantization (LVQ), a prototype-based supervised classification algorithm. Web page https://en.wikipedia.org/w/index.php?title=Cosine_similarity&oldid=411040778 "Cosine Similarity" relates to Cosine similarity, a measure of similarity between two vectors by measuring the cosine of the angle between them. Patent application publication US 2012/0040332 A1 relates to methods, assays and kits for detecting or monitoring adrenal tumours by detecting one or more adrenal steroid metabolites., the metabolites selected from THS (tetrahydro-11-desoxycortisol), 5-PT (5-pregnenetriol), 5-PD (pregnenediol), PD (pregnanediol), PT (pregnanetriol), Et (etiolanolone), TH-DOC (tetrahydro-11-corticosterone), (5α-THA) 5α-tetra-11-hydrocorticosterone, and 5α-THF (5α-tetra hydrocortisol). "Urine Steroid Metabolomics as a Biomarker Tool for Detecting Malignancy in Adrenal Tumors", Wiebke Arlt et al., Journal of clinical Endocrinology & Metabolism, vol. 96, no. 12, 1 December 2011 pages 3775-3784, relates to detection of adrenal malignancy using a steroid metabolomic approach, including mass spectrometry-based steroid profiling followed by machine learning analysis.

The present invention provides a computer program for identifying a disease state, a computer readable medium, and an electronic device as set out in appended claims 1, 7 and 8. The application describes a novel interpretable machine learning method for the computer-aided diagnosis of three conditions including the most prevalent, 21-hydroxylase deficiency (CYP21A2), and two other representative, but rare conditions, 5α-reductase type 2 deficiency (SRD5A2) and P450 oxidorectase deficiency (PORD). The data set contains a large collection of steroid metabolomes from over 800 healthy controls of varying age (including neonates, infants, children, adolescents and adults) and over 100 patients with newly diagnosed, genetically confirmed inborn steroidogenic disorders.

The data set and problem formulation comprises several computational difficulties. On average 8% to 13% of measurements from healthy control and patients respectively are missing or not detectable. The problem now arises because those measurements are not missing at random but systematically, since the data collection combines different studies and quantitation philosophy has changed over the years. Furthermore, the measurements are very heterogeneous. Neonates and infants naturally deliver less urine, with usually only a spot urine or nappy collection available, rather than an accurate 24-h urine. Moreover, the individual excretion amounts vary a lot due to maturation-dependent, natural adrenal development and peripheral factors; this affects even healthy controls but much more so patients with steroidogenic enzyme deficiencies. Moreover, some disease conditions are rare which poses an insuperable obstacle for state-of-the-art imputation methods for the missing values. To account for these difficulties the invention provides an interpretable prototype-based machine learning method using a dissimilarity between two metabolomic profiles based on the angle $\Theta$ between them calculated on the observed dimensions.

Using the angles instead of distances has two principal advantages: (1) distances calculated in spaces of varying dimensionality (depending on the number of shared observed dimensions in two metabolomic fingerprints) do not share the same scale and (2) the angles naturally express the idea that only the *proportional* characteristics of the individual profiles matter.

The same approach may be used to identify or detect the disease states and a number of other different other diseases, by measuring the metabolic data from subjects. These diseases might include for example, diseases caused by bacterial or viral infections, and also additionally metabolic or endocrine diseases.

A first aspect of the invention provides a method of characterising a disease state comprising:

(i) collecting metabolic data from a plurality of subjects;

(ii) presenting the data as vectors with dimensions corresponding to different biomarkers: and

(iii) weighting the importance of either individual dimensions, or the interplay among multiple dimensions when calculating angles of the vectors, such that there is a minimum variation of angle within a disease class and/or a maximum variation of angle compared to a different disease class.

The weighting in step (iii) may be global (for all diseases) or local (specific for each disease state).

This identifies those biomarkers which are characteristic of the disease state.

Metabolic data may be obtained from a variety of different sources, including for example, tissue samples, blood, serum, plasma, urine, saliva, tears or cerebrospinal fluid. The sample may be analysed by any techniques generally known in the art to obtain the presence of, or amount of different compounds within that sample.

For example, the presence of a concentration or amount of different compounds may be determined by, for example, chromatography or mass spectrometry, such as gas chromatography-mass spectrometry or liquid chromatography-tandem mass spectrometry. This includes, for example, uPLC tandem mass spectrometers, which may be used in positive ion mode. This is described in, for example, WO 2010/092363.

This is known as metabolic data as it shows metabolites within the sample.

The data is then presented as vectors with dimensions corresponding to different biomarkers or compounds. Typically the method uses one or more prototype vectors for each class. These can be initialized randomly, close to the mean vector of the group or can be provided by an expert as an estimate of the likely typical vector. The algorithm will adapt the weighting of biomarkers during training. This allows, for example, commonly occurring biomarkers with little relevance to the disease state to be discounted. During training the prototypes and relevance matrix/matrices are compared to data from individuals with known disease states and changed in order to minimise the variation of angle between the disease class and simultaneously maximise the variation of angle between different disease classes.

Typically the applicant provides 3 levels of complexity depending on the number of parameters trained on. The weighting influence may be:

1. individual dimensions
2. additionally pairwise correlated dimensions via full metric tensor
3. localized metric tensors for each of the classes

The description below shows a typical formula used. In summary the form of the matrix R makes the difference, for 1. It is a diagonal matrix containing a vector of relevances, for 2. it is a matrix product of $AA^t$ and for 3. there are local matrices $R^c$ attached to the prototypes

Metabolic data of a subject can then be compared to the trained prototypes and relevance matrix/matrices to identify the presence of, or follow progression of a disease state in that subject. Besides this analytical analysis the method may provide visualisations for interpretable access to the model.

The method further provides comparing the trained prototype and optionally the relevance matrix/matrices to metabolic data of a subject, to identify the presence of, or follow progression of, a disease state in that subject.

The metabolic data of that subject may be presented as vectors with dimensions corresponding to different biomarkers which then may be compared to the prototype and optionally the relevance matrix/matrices, to identify the presence or absence of the disease state or follow progression of the disease state.

Methods of identifying a disease state or following progression of a disease state of a subject, is also provided comprising a method of identifying a disease state comprising:

(i) collecting metabolic data from the subject;

(ii) presenting the data as vectors with dimensions corresponding to different biomarkers: and

(iii) comparing two or more angles of vectors with a prototype vector and optionally at least one relevance matrix, to identify the presence of, or progression of, a disease state.

In a preferred aspect of the invention, the vector of a precursor biomarker is compared to a vector of a metabolite of the precursor biomarker.

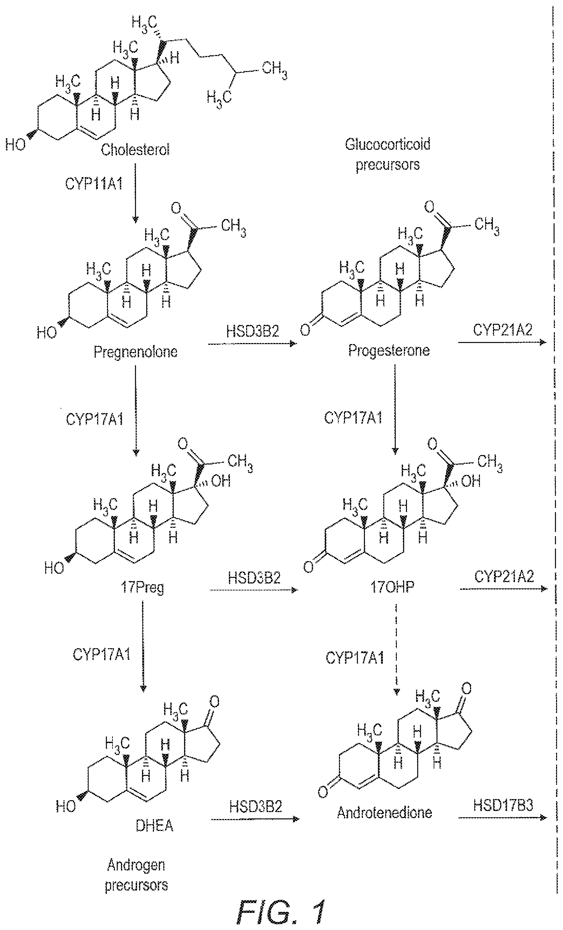

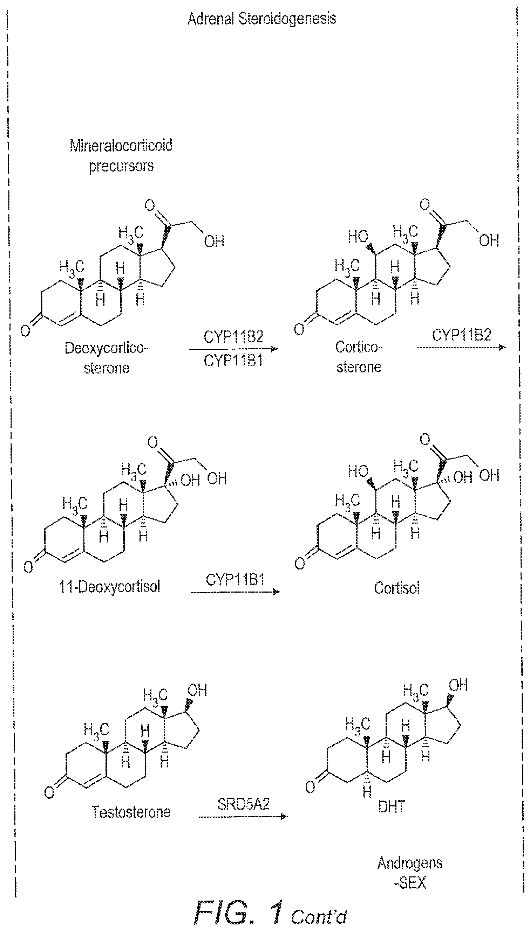

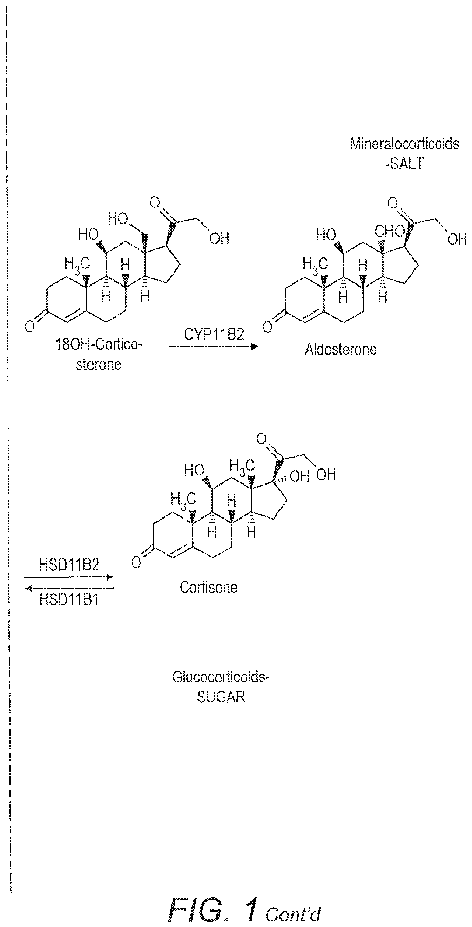

For example, Figure 1 shows adrenal steroidogenesis. A number of different diseases are associated with abnormalities in this pathway. These may be due to, for example, the altered function of a particular enzyme, which converts a precursor into a metabolite or mutations in such enzymes which affect the amount of metabolite produced. The diseases are usually accompanied by an excess of the pathway parts which are not affected by the deficiency because of the tailback of precursors. That excess in other parts of the pathway might however be individually different, which makes the problem complicated for manual analysis.

Accordingly, a precursor may be, for example, pregnenolone. A mutation or deletion of the enzyme CYP17A1 might result in a difference in the relative amounts or ratios of 17PREG or DHEA produced as metabolites. Alternatively, there may be a mutation in the pathway that produces aldosterone or cortisone. Accordingly, the metabolites compared with the pregnenolone precursor may be, for example, corticosterone or aldosterone or alternatively a member of the cortisone pathway such as cortisol or cortisone. Similarly, 11-deoxycortisol may be used as a precursor biomarker and compared to, for example, cortisol or cortisone to identify mutations in that part of the pathway. In unclaimed examples, similar analysis may be carried out in other complex pathways having a number of different metabolites to identify other metabolic or endocrine disease.

The disease state may be a metabolic disease state or an endocrine disease state. Alternatively, this may be used as a marker, for example, for a tumour, where the tumour produces a number of different metabolites. The disease is a disease affecting steroidogenesis. Such conditions include inborn steroidogenic disorders, with inactivating mutations in CYP21A2, CYP17A1, CYP11B, HSD3B2, POR, SRD5A2 and HSD17B3 resulting in a combination of adrenal insufficiency and disordered sex development. Similarly, the differentiation of benign from malignant adrenal tumours and the differentiation of different hormone excess states in both benign and malignant adrenal tumours may be aided by the method, which would similarly apply to other tumours of steroidogenically competent tissue e.g. arising from the gonads.

Methods of the invention may be used to identify a disease fingerprint which is diagnostic of the disease. That is, the method produces an indication of the markers, the presence or absence of which, is associated with the disease state. The presence or absence of those disease markers may be determined by alternative methods of detecting those markers. For example, the method may identify that the presence of two or three specific markers associated with the disease state. The markers may then be detected by an alternative detection system, for example, an immunoassay.

The diseases or conditions found or monitored can then be treated by a physician, for example, using treatments generally known in the art for the disease or conditions.

Computer implemented methods of detecting a disease state, following progression of a disease state or providing a fingerprint of a disease state comprising collecting metabolic data and performing the methods according to the invention, followed by transmitting information to a user of the disease state or the fingerprint are also provided. Computer readable medium instructions which when performed carry out the method of the invention are similarly provided.

Electronic devices having a precursor and a memory, the memory storing instructions which when carried out cause a precursor processor to carry out the method of the invention and transmit information regarding the disease state or fingerprint to the user, are also provided.

The methods utilised in the invention are generally known as Angle Learning Vector Quantization (Angle LVQ or ALVQ). This typically uses cosine dissimilarity instead of Euclidean distances. This makes the LVQ variant robust for classification of data containing missingness.

The method typically used is as follows.

We propose Angle Learning Vector Quantization (angle LVQ) as an extension to Generalized LVQ (GLVQ) and variants [4, 3, 5]. As in the original formulation we assume training data given as z-transformed vectorial measurements (zero mean, unit standard deviation) accompanied by labels $\{(x_i, y_i)\}_{i=1}^{N}$ and a user determined number of labelled protoypes $\{(w_m, c(w_m))\}_{m=1}^{M}$ representing the classes. Classification is performed following a Nearest Prototype Classification (NPC) scheme, where a new vector is assigned the class label of its closest prototype.

Our approach differs from GLVQ by using an angle based similarity instead of the Euclidean distance. Both prototypes and relevances R are determined by a supervised training procedure minimizing the following cost function [7] calculated on the observed dimensions:

$$E = \sum_{i=1}^{N} \frac{d_i^J - d_i^K}{d_i^J + d_i^K}$$

Here the dissimilarity of each data sample $x_i$ with its nearest correct prototype with $y_i = c(w_J)$ is defined by $d_i^J$

and by $d_i^K$ for the closest wrong prototype ($y_i \neq c(w_K)$). Now distances $d_i^{\{J,K\}}$ are replaced by angle-based dissimilarities:

$$d_i^L = g_\beta \left( \frac{x_i R w_L^T}{\sqrt{(x_i R x_i^T)}\sqrt{w_L R w_L^T}} \right) \qquad (1)$$

$$\text{With } g_\beta(b) = \frac{\exp\{-\beta(b-1)\}-1}{exp(2\beta)-1} \quad \text{and } L\epsilon\{J,K\} \qquad (2)$$

Here, the exponential function $g_\beta$ with slope $\beta$ transforms the weighted dot product b = cos $\Theta_R \in$ [-1, 1] to a dissimilarity $\in$ [0, 1]. Finally, training is typically performed by minimizing the cost function E, which exhibits a large margin principle [4].

Dependent on the parametrization of the dissimilarity measured the complexity of the algorithm can be changed. In the case of R being the identity matrix the algorithm adapts the prototypes only. With *R=diag(R)* additionally to the prototypes the relevance of each dimension $\{r_j\}_{j=1}^D$ can be adapted. In case of $R = AA^T$ with $A = \mathbb{R}^{Dxb}$ for $b \leq D$ a linear transformation to the b-dimensional space is learned which is able to weight not only individual dimensions $A_{ii}$, but also pairwise correlations of dimensions $A_{ij}$. The most complex version of the algorithm introduces local dissimilarity measures $R_c = A_c A_c^T$ ($A_c \in \mathbb{R}^{D \times b_c}$) attached to prototypes $w_c$, which can adapt relevant dimensions important for the classification of individual classes.

### A. Relevance vector version of angle LVQ

To ensure positivity of the relevances we set $r_j = a_j^2$ and we optimize $a_j$'s collected in a vector *a*. We furthermore restrict r by a penalty term $(1 - \Sigma_j r_i)^2$ added to E. Lastly we added a regularization term $-\gamma \Sigma_j \log r_j$ to E to prevent oversimplification effects. Optimization can be performed for example by steepest gradient descent. The derivatives of equation 1 with $R_{jj} = a_j^2$ and $\|v\|_A = \sqrt{\sum_{m=1}^M v_m^2 a_m^2}$ are

$$\frac{\partial E}{\partial w_J} = \sum_{i=1}^N \frac{2d_i^K}{\left(d_i^J + d_i^K\right)^2} \frac{\partial d_i^J}{\partial w^J} \quad \text{and} \quad \frac{\partial E}{\partial w_K} = \sum_{i=1}^N \frac{-2d_i^K}{\left(d_i^J + d_i^K\right)^2} \frac{\partial d_i^J}{\partial w^J} \qquad (3)$$

$$\frac{\partial g_\beta(b)}{\partial b} = -\frac{-\beta\exp\{-\beta b + \beta\}}{\exp\{2\beta\}-1} \qquad (4)$$

$$\frac{\partial d^L}{\partial w_{\{L,j\}}} = \frac{\partial g_\beta}{\partial w_L} \frac{a_j^2(x_j \sum_m w_{\{L,m\}}^2 a_m^2 - \sum_m x_m w_{\{L,m\}} a_m^2)}{\|x\|_A \|w_L\|_A^3} \qquad (5)$$

$$\frac{\partial E}{\partial a_j} = \sum_{i=1}^N \frac{2d_i^K \frac{\partial d_i^J}{\partial a_j} - 2d_i^J \frac{\partial d_i^K}{\partial a_j}}{\left(d_i^J + d_i^K\right)^2} \qquad (6)$$

$$\frac{\partial d^L}{\partial a_j} = \frac{a_j 2 x_j w_{\{L,j\}}}{\|x\|_A \|w_L\|_A} - x_j^2 \sum_m \frac{x_m w_{\{L,m\}} a_j^2}{\|x\|_A^3 \|w_L\|_A} - \frac{w_j^2 \sum_m x_m w_{\{L,m\}} a_m^2}{\|x\|_A \|w_L\|_A^3} \qquad (7)$$

### B. Relevance matrix version angle LVQ

A similar extension of Generalized Matrix LVQ(GMLVQ)[5] we now use $R = AA^T$ in the angle based similarity

$$d_i^{\{J,K\}}:$$

$$d_i^L = g_\beta \left( \frac{(x_i AA^T w_L^T)}{\sqrt{x_i AA^T x_i^T} \sqrt{w_L AA^T w_L^T}} \right) \tag{8}$$

The derivatives of E (Eq 1) with $\|v\|_A = \sqrt{(vAA^Tv)}$ are:

$$\frac{\partial d^L}{\partial w_{L,}} = \frac{\partial g_\beta}{\partial w_L} \frac{xAA^T\|w_L\|_A^2 - xAA^T w_L . w_L AA^T)}{\|x\|_A \|w_L\|_A^3} \tag{9}$$

$$\frac{\partial E}{\partial A_{md}} = \frac{x_m \sum_j A_{jd} w_{\{L,j\}} + w_{\{L,m\}} \sum_j A_{jd} x_j}{\|x\|_A \|w_L\|_A} - xAA^T w_L. \tag{9}$$

$$\left[ \frac{x_m \sum_j A_{jd} x_j}{\|x\|_A^3 \|w_L\|_A} + \frac{w_{\{L,m\}} \sum_j A_{jd} w_{\{L,j\}}}{\|x\|_A \|w_L\|_A^3} \right] \tag{10}$$

Where $v_{\{.,j\}}$ denotes dimension j of vector v.

## C. Local relevance matrix version of angle LVQ

As proposed in Limited Rank Matrix LVQ we now use $R_C = A_C A_C^T$ in the angle based similarity $d_i^{\{J,K\}}$:

$$d_i^c = g_\beta \left( \frac{(x_i AA^T w_L^T)}{\sqrt{x_i A_c A_c^T x_i^T} \sqrt{w_c A_c A_c^T w_c^T}} \right) \tag{11}$$

$$\frac{\partial d^c}{\partial w_{c,}} = \frac{\partial g_\beta}{\partial w_c} \frac{x A_c A_c^T \|w_c\|_{A_c}^2 - x A_c A_c^T w_c . w_c A_c A_c^T)}{\|x\|_{A_c} \|w_c\|_{A_c}^3} \tag{12}$$

$$\frac{\partial E}{\partial A_{\{c,md\}}} = \frac{x_m \sum_j A_{\{c,jd\}} w_{\{c,j\}} + w_{\{c,m\}} \sum_j A_{\{c,jd\}} x_j}{\|x\|_{A_c} \|w_c\|_{A_c}} - x A_c A_c^T w_c.$$

$$\left[ \frac{x_m \sum_j A_{\{c,jd\}} x_j}{\|x\|_{A_c}^3 \|w_c\|_{A_c}} + \frac{w_{\{c,m\}} \sum_j A_{\{c,jd\}} w_{\{c,j\}}}{\|x\|_{A_c} \|w_c\|_{A_c}^3} \right] \tag{13}$$

Where $v_{\{.,ij\}}$ denotes dimension ij of matrix V.

In order to handle the imbalanced classes, a modification may be made to angle LVQ, refered to henceforth as cost-defined angle LVQ. Here explicit costs [6] was introduced so as to boost learning to differentiate between disease classes (all minority classes) and the healthy class (majority class).

We introduced a hypothetical cost matrix G=$\gamma_{cp}$, with $\Sigma^c \gamma_{cp}$ =1. The rows correspond to the actual classes c and columns denote the predicted classes p. We include those costs in our cost function

Eq.(1),

$$\hat{E} = \sum_{i=1}^{N} \frac{\gamma_c p(\underline{x}_i)}{n_c} \mu(\tilde{x}_i)$$

where c = yi is the class label of sample $\tilde{x}_i$, $n_c$ defines the number of samples within that class and $p$ being the predicted label (label of the nearest prototype). These hypothetical costs were highest for the most dangerous misclassification (misclassifying a patient to healthy), and for the correct classifications. The images above illustrate how the penalization scheme appears. The higher the cost, the greater the penalization for misclassification and reward for correct classification.

As a preferred alternative approach to dealing with imbalanced class problem, we tried oversampling of the minority samples. In this approach new training samples are artificially synthesized to increase the minority class. We have made and applied, for example, a variant of the original Synthetic Minority Over-sampling Technique (SMOTE) (proposed in [6]) which synthesized samples on the hypersphere (so adjust for the fact that angle LVQ classifies on the hypersphere). For this we used an important tool of Riemannian geometry, which is the exponential map [7, 8]. The exponential map has an origin M which defines the point for the construction of the tangent space $T_M$ of the manifold. Let P be a point on the manifold and $\hat{P}$ a point on the tangent space then P = $Log_M P$, P = $Ex_{PM}\hat{P}$ and $d_g(P, M) = d_e(\hat{P}, M)$ with $d_g$ being the geodesic distance between the points on the manifold and $d_e$ being the Euclidean distance on the tangent space. The Log and Exp notations denote a mapping of points from the manifold to the tangent space and vice versa. In our case we present a point $\tilde{x}$ from class c on the unit sphere with fixed length $|\tilde{x}| = 1$, which becomes the origin of the map and the tangent space (the centre of the hypersphere is the origin). We find k nearest neighbours $\tilde{x}_\psi \in N_{\tilde{x}}$ of the same class as selected sample $\tilde{x}$ using the angle between the vectors $\theta = \cos^{-1}$ ($\tilde{x} > \tilde{x}_\psi$). Each random neighbour $\tilde{x}_\psi$ is now projected onto that tangent space using only the present features and the $Log_M$ transformation for spherical manifolds:

$$\widehat{\tilde{x}_\psi} = \frac{\theta}{(sin)\theta}(\tilde{x}_\psi - \tilde{x}\,cos\theta)$$

Next, a synthetic sample is produced on the tangent space as before $\hat{s} = \tilde{x} + \alpha \cdot (\hat{x}_\psi - x)$. The new angle $\hat{\theta} = |\hat{s}|$ is then used to project the new sample back to the unit hypersphere by the $Exp_M$ transformation:

$$\hat{s} = \tilde{x}\cos\hat{\theta} + \frac{sin\hat{\theta}}{\hat{\theta}}\hat{\tilde{s}}$$

(16)

This procedure is repeated with another sample from the class until the desired number of training samples is reached for that class.

For convenient visualization of 3 dimensional globe (on which the data from the different classes are plotted) Mollweide projection was typically used to flatten out the sphere into a map. Mollweide projection is given by

$$x = \frac{R2\sqrt{2}}{\pi}(\lambda - \lambda_0)\cos\theta$$

$$y = R\sqrt{2}\sin\theta$$

$$\theta_{n+1} = \theta_n - \frac{2\theta_n + \sin2\theta_n - \pi\sin\phi}{2 + 2\cos2\theta_n}$$

$$\theta_0 = \phi$$

The invention will now be described by way of example only, with reference to the following figures:

**Figure 1** shows the adrenal steroidogensis pathway.

**Figure 2** shows the variability of different metabolites which are secreted by heathly individuals showing the complexity of the numbers of different compounds produced by heathly individuals.

**Figure 3** shows that the secretion of a number of different steroids is very variable with the age of the individual.

**Figure 4** shows the original 35 metabolite fingerprint dimensions representation.

**Figure 5** shows a representation of vectors for 165 dimensions build using problem specific expert knowledge and ANOVA.

**Figure 6** shows an example relevance matrix for angle LVQ found by cross-validation. Dark regions in the Relevance matrix R figure indicate important pairwise dimensions of ratios and white less important ones.

**Figure 7** shows an example 2D visualisation of the relevance matrix angle LVQ for different conditions. CYP21A2 (squares), POR (triangles) and SRD5A2 (circles) compared to prototypes (star) and healthy (dots). The diamonds correspond to some typical examples from each condition.

**Figure 8** shows relevance vector of an example angle LVQ model found by cross validation.

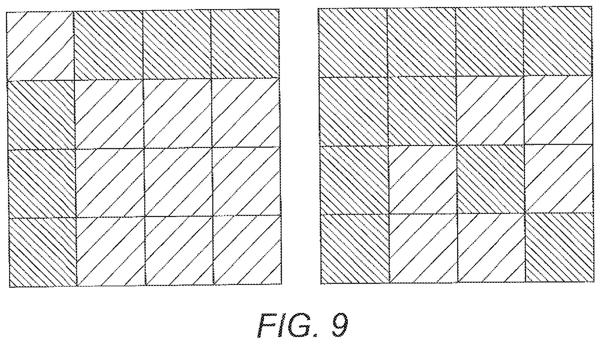

**Figure 9** shows representation of cost definitions using cost-defined angle LVQ. The dark blocks correspond to higher cost definitions.

**Figure 10** shows Boxplots showing perfomance criteria for local LVQ with a feature set (setting S8) and reduced feature set exemplified in Table 1 below; a) perfomance of the classifier for each of the performance settings during training; b) the performance of the classifier for each of the specific settings during validation; c) the performance of the classifier for each of the specific settings during generalisation.

**Figure 11** shows projection of data classified by ALVQ global matrix with dimension 2 and 3: a) Projection of data prints one of the models of ALVQ with 2D global matrix with cost definitions; b) 3D projection with cost dimensions.

**Figure 12** costs projection of classified data on a sphere and its corresponding map projection: a) projection of data classified by one of the models of ALVQ with 3D global matrix of cost projections in b) in map projection; c) projection of data (seen and unseen) classified by one of the models of ALVQ with 3D global matrix with cost projections and d) in map projection.

**Figure 13** shows visualisation of 6-class classification by geodesic SMOTE (100% oversampling) coupled with ALVQ with $\beta=1$ dimension = 3, global matrix: a) projection of data prints from classification by one of the models of ALVQ with 2D global matrix and b) Mollweide proejction; c) projection of only the data prints from the classification by the model used in a) for easier visualisation.

**Figre 14** shows boxplots for the performance criteria described below for the local ALVQ with full feature set for 4 class problem and 6 class problem; a) the performance of the classifier for each of the specified settings during training; b) the performance of the classsified for each of the specified setting during validation.

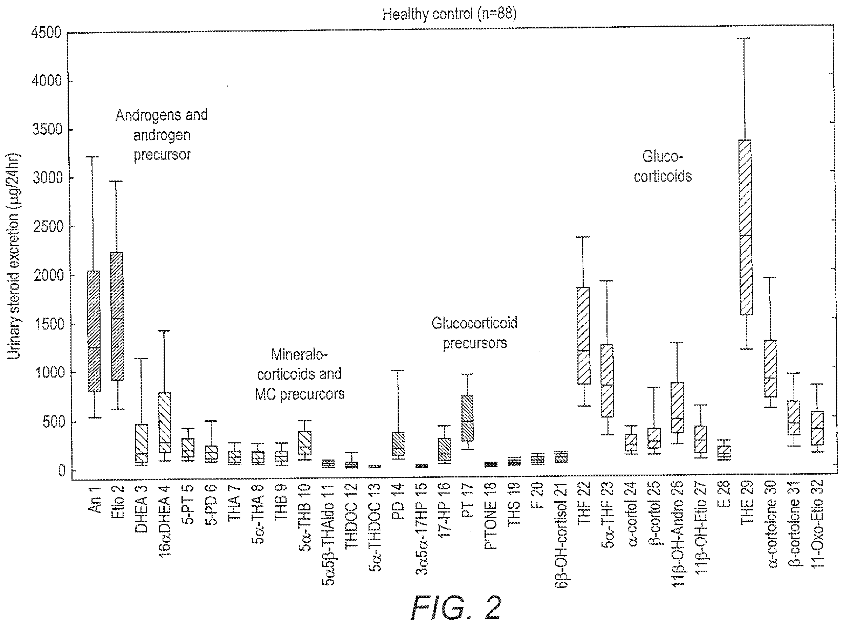

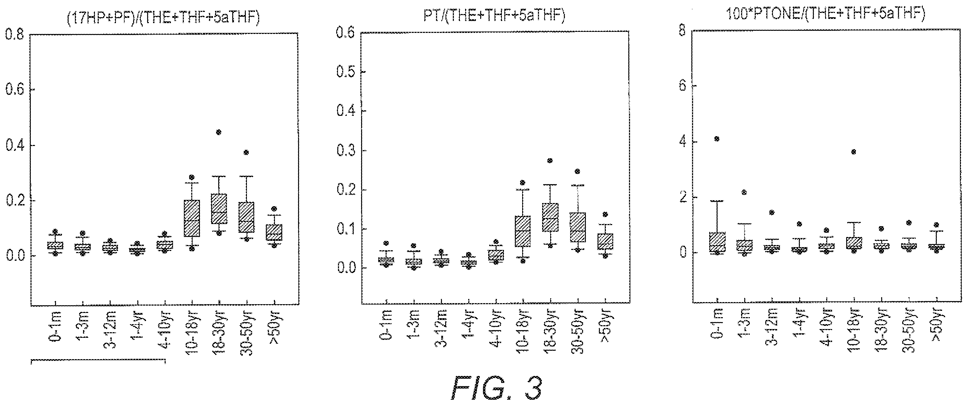

Figure 2 shows that a variety of metabolites which are secreted by healthy individuals and Figure 3 shows they are produced in different amounts depending on age of the subject. This demonstrates the complexity of this data domain and demonstrates some of the problems which the Applicant sought to overcome

In the Example, urine samples were measured and in the prototype the applicant started to work with the 34dim vector of metabolites acquired by automatic quantitation of the spectrum.

In the first experiments the starting dimension corresponded to:

ANDROS, ETIO, DHEA, $16\alpha$-OH-DHEA, 5-PT, 5-PD, Pregnadienol, THA, $5\alpha$ -THA, THB, $5\alpha$-THB, $3\alpha5\beta$-THALDO, TH-DOC, $5\alpha$-TH-DOC, PD, $3\alpha5\alpha$-17HP, 17HP, PT, PTONE, THS, Cortisol, $6\beta$-OH-F, THF, $5\alpha$-THF, $\alpha$-cortol, $\beta$-cortol, $11\beta$-OH-AN, $11\beta$-OH-ET, Cortisone, THE, $\alpha$-cortolone, $\beta$-cortolone, 11-OXO-Et, 18-OH-THA, These correspond to metabolites in the Adrenal steroidogenesis pathway summarised in Figure 1.

| No. | Abbreviation | Common name | Chemical name | Metabolite of |
|---|---|---|---|---|
| **Androgen metabolites** | | | | |
| 1 | An/ANDROS | Androsterone | 5α-androstan-3a-ol-17-one | Androstenedione, testosterone, 5a-dihydrotestosterone |
| 2 | Etio | Etiocholanolone | 5β-androstan-3a-ol-17-one | Androstenedione, testosterone |
| **Androgen precursor metabolites** | | | | |
| 3 | DHEA | Dehydroepiandrosterone | 5-androsten-3β-ol-17-one | DHEA + DHEA sulfate (DHEAS) |
| 4 | 16α-OH-DHEA | 16α-hydroxy-DHEA | 5-androstene-3β,16α-diol-17-one | DHEA + DHEAS |
| 5 | 5-PT | | 5-pregnene-3β,17, 20α-triol | |
| 6 | 5-PD | | 5-pregnene-3β, 20α-diol and 5, 17, (20)-pregnadien-3β-ol | Pregnenolone |
| **Mineralocorticoid metabolites** | | | | |
| 7 | THA | Tetrahydro-11-dehydrocorticosterone | 5β-pregnane-3α, 21-diol, 11, 20-dione | Corticosterone, 11-dehydrocorticosterone |
| 8 | 5α-THA | 5α-tetrahydro-11-dehydrocorticosterone | 5α-pregnane-3α, 21-diol-11, 20-dione | Corticosterone, 11-dehydrocorticosterone |
| 9 | THB | Tetrahydrocorticosterone | 5β-pregnane-3α, 11β, 21-triol-20-one | Corticosterone |
| 10 | 5α-THB | 5α-tetrahydrocorticosterone | 5α-pregnane-3α, 11β, 21-triol-20-one | Corticosterone |
| 11 | 3α5β-THALDO | Tetrahydroaldosterone | 5β-pregnane-3α, 11β, 21-triol-20-one-18-al | Aldosterone |
| **Mineralocorticoid precursor metabolites** | | | | |
| 12 | THDOC | Tetrahydro-11-deoxycorticosterone | 5β-pregnane-3α, 21-diol-20-one | 11-deoxycorticosterone |
| 13 | 5α-THDOC | 5α-tetrahydro-11-deoxycorticosterone | 5α-pregnane-3α, 21-diol-20-one | 11-deoxycorticosterone |
| **Glucocorticoid precursor metabolites** | | | | |
| 14 | PD | Pregnanediol | 5β-pregnane-3α, 20a-diol | Progesterone |
| 15 | 3α5α-17HP | 3α, 5α-17-hydroxy-pregnanolone | 5α-pregnane-3α, 17α-diol-20-one | 17-hydroxy-progesterone |
| 16 | 17HP | 17-hydroxy-pregnanolone | 5β-pregnane-3α, 17α,-diol-20-one | 17-hydroxy-progesterone |
| 17 | PT | Pregnanetriol | 5β-pregnane-3α, 17α, 20α-triol | 17-hydroxy-progesterone |
| 18 | PTONE | Pregnanetriolone | 5β-pregnane-3α, 17, 20α-triol-11-one | 21-deoxycortisol |
| 19 | THS | Tetrahydro-11-deoxycortisol | 5β-pregnane-3α, 17, 21-triol-20-one | 11-deoxycortisol |
| **Glucocorticoid metabolites** | | | | |
| 20 | F | Cortisol | 4-pregnene-11β, 17, 21-triol-3, 20-dione | Cortisol |
| 21 | 6β-OH-F | 6β-hydroxy-cortisol | 4-pregnene-6β, 11β, 17, 21-tetrol-3, 20-dione | Cortisol |

(continued)

**Glucocorticoid metabolites**

| 22 | THF | Tetrahydrocortisol | 5β-pregnane-3α, 11β, 17, 21-tetrol-20-one | Cortisol |
|---|---|---|---|---|
| 23 | 5α-THF | 5α-tetrahydrocortisol | 5α-pregnane-3α, 11β, 17, 21-tetrol-20-one | Cortisol |
| 24 | α-cortol | α-cortol | 5α-pregnan-3α, 11β, 17, 20β, 21-pentol | Cortisol |
| 25 | β-cortol | β-cortol | 5β-pregnan-3α, 11β, 17, 20β, 21-pentol | Cortisol |
| 26 | 11b-OH-An | 11β-hydroxy-androsterone | 5α-androstane-3α, 11β-diol-17-one | Cortisol (+ Androgens) |
| 27 | 11b-OH-Et | 11b-hydroxy-etiocholanolone | 5β-androstane-3α, 11β-diol-17-one | Cortisol (+ Androgens) |
| 28 | E | Cortisone | 4-pregnene-17α, 21-diol-3, 11, 20-trione | Cortisol |
| 29 | THE | Tetrahydrocortisone | 5β-pregnene-3α, 17, 21-triol-11, 20-dione | Cortisol |
| 30 | α-cortolone | α-cortolone | 5β-pregnane-3α, 17, 20α, 21-tetrol-11-one | Cortisol |
| 31 | β-cortolone | β-cortolone | 5β-pregnane-3α, 17, 20β, 21-tetrol-11-one | Cortisol |
| 32 | 11-oxo-Et | 11-oxo-etiocholanolone | 5β-androstan-3α-ol-11, 17-dione | Cortisol (+ Androgens) |

Typical examples for the disease types:

Record Nb 470 Age 18.00 condition Healthy:
482.63, 815.52, 56.03, 176.66, 143.00, 107.09, NaN, 76.43, 41.25, 73.64, 132.85, NaN, NaN, NaN, 149.15, NaN, 64.21, 205.22, 4.90, 43.31, 29.31, NaN, 705.63, 421.75, 114.99, 246.09, 225.67, 214.90, 36.17, 2051.85, 716.78, 307.66, 497.61, NaN,

Record Nb 391 Age 2.56 condition Healthy:
5.00, 5.00, 9.00, 8.00, 8.00, 57.00, 23.00, 33.00, 35.00, 30.00, 70.00, 33.00, 1.00, 8.00, 9.00, 1.00, 17.00, 14.00, 1.00, 28.00, 20.00, 38.00, 193.00, 327.00, 11.00, 134.00, 21.00, 7.00, 28.00, 693.00, 42.00, 121.00, 16.00, 1530.00,

Record Nb 881 Age NaN condition CYP21A2:
222.00, 17.00, 100.00, 20187.00, 50.00, 599.00, 1034.00, 128.00, 0.00, 0.00, 0.00, 75.00, 341.00, 115.00, 102.00, 127.00, 628.00, 292.00, 521.00, 49.00, 122.00, 257.00, 130.00, 224.00, 240.00, 112.00, 498.00, 45.00, 788.00, 80.00, 13.00, 220.00, 545.00, 0.00,

Record Nb 895 Age 16.45 condition POR:
553.50, 769.50, 230.00, 15.00, 1089.00, 4607.00, 7403.00, 1466.00, 225.50, 451.50, 1038.50, 21.00, 146.00, 34.00, 4523.00, 94.50, 1877.50, 3923.00, 504.50, 89.50, 60.50, 7.50, 663.50, 390.00, 27.50, 298.50, 165.50, 81.00, 43.50, 5101.00, 423.00, 720.50, 188.50, 194.00,

Record Nb 917 Age 7.75 condition SRD5A2:
83.00, 446.00, 326.00, 19.00, 119.00, 389.00, 47.00, 342.00, 17.00, 253.00, 232.00, NaN, 14.00, 52.00, 166.00, 2.00, 71.00, 306.00, 8.00, 120.00, 94.00, 184.00, 1076.00, 9.00, 89.00, 281.00, 85.00, 184.00, 111.00, 4044.00, 962.00, 521.00, 321.00, 106.00,

From these samples we build ratio vectors by upstream pathway grouping of metabolites to reduce the $34^2$ possibilities followed by ANOVA for each condition vs healthy:
This leads to 165 potential interesting ratios of the original metabolites:
THS/Cortisol, THS/Cortisone, ANDROS/11β-OH-ANDRO, THS/11β-OH-ANDRO, THS/PT-ONE, THS/6β-OH-F, 5-PT/PT-ONE, TH-DOC/Cortisol, TH-DOC/PT-ONE, TH-DOC/Cortisone, 5-PT/Cortisol, PT/PT-ONE, 5-PT/Cortisone, TH-DOC/6-β-OH-F, ETIO/11β-OH-ANDRO, 5-PT/11β-OH-ANDRO, PT/11β-OH-ANDRO, TH-DOC/11β-OH-ANDRO, PD/PT-ONE, DHEA/11β-OH-ANDRO, 18-OH-THA/16α-OH-DHEA, PT-ONE/16α-OH-DHEA, PD/11β-OH-ANDRO, 5-PT/6β-OH-F, PT/Cortisol, THS/16α-OH-DHEA, 18-OH-THA/6β-OH-F, 3a5β-THALDO/16α-OH-DHEA, 18-OH-THA/Cortisone, Cortisol/16α-OH-DHEA, 18-OH-THA/Cortisol, β-cortolone/16α-OH-DHEA, PT/β-cortol, PT/β-cortolone, PT/THE, 11-OXO-Et/THE, PT/THF, PT/5-α-THF, THE/11-β-OH-ANDRO, β-cortol/11β-OH-ANDRO, TH-DOC/THE, PT-ONE/-β-cortol, PT-ONE/-β-cortolone, PT-ONE/THE, THE/ANDROS, PT-ONE/5α-THF, PT-ONE/THF, PT/6β-OH-F, PT-ONE/α-cortol, PTONE/α-cortolone, PT-ONE/6β-OH-F, PT-ONE/11β-OH-ANDRO, TH-DOC/β-cortolone, 5α-THA/PT, 5α-THA/PT-ONE, THA/PT-ONE, PT-ONE/ANDROS, PT-ONE/11β-OH-ETIO, 18-OH-THA/PT, 18-OH-THA/PT-ONE, TH-DOC/5-α-THF, PD/THE, TH-DOC/α-cortolone, 17-HP/β-cortol, 17-HP/α-cortolone, 17-HP/THE, 17-HP/β-cortolone, 17-HP/THF, 17-HP/5α-THF, 17-HP/α-cortol, 17-HP/THS, TH-DOC/18-OH-THA, 5α-THA/17-HP, 17-HP/6β-OH-F, 17-HP/ANDROS, Cortisone/11-β-OH-ANDRO, TH-DOC/β-cortol, 5-α-THF/11β-OH-ANDRO, PT/ANDROS, TH-DOC/5α-THA, THF/11-β-OH-ANDRO, 17-HP/11-β-OH-ANDRO, 18-OH-THA/17-HP, 17-HP/PT-ONE, PT-ONE/11-OXO-Et, 11-OXO-Et/β-cortolone, TH-DOC/α-cortol, 18-OH-THA/11β-OH-ANDRO, TH-DOC/THF, 5-PT/THE, PTONE/Cortisol, 17-HP/11-β-OH-ETIO, PT/α-cortolone, 5α-THB/α-cortolone, THA/5-PT, 5-PT/THS, 18-OH-THA/α-cortolone, 18-OH-THA/THE, TH-DOC/THS, TH-DOC/3α5β-THALDO, 18-OH-THA/THF, THB/17-HP, THB/PT-ONE, THF/11-OXO-Et, PT/Cortisone, Cortisone/16α-OH-DHEA, THA/16α-OH-DHEA, THB/5-PT, β-cortolone/11β-OH-ANDRO, 5α-THB/α-cortol, PT/α-cortol, 17-HP/DHEA, 5-PT/DHEA, PT/DHEA, β-cortol/DHEA, PD/17-HP, THA/17-HP, THA/11β-OH-ANDRO, 5-PT/β-cortolone, TH-DOC/5-PT, PT/11β-OH-ETIO, 5α-THB/5-PT, THB/11β-OH-ANDRO, THA/α-cortol, THA/α-cortolone, 5α-TH-DOC/3a5β-THALDO, THB/PT, THA/Cortisone, 18-OH-THA/5α-THF, 5α-THB/5α-THF, THS/DHEA, THE/DHEA, β-cortolone/DHEA, THA/β-cortolone, PD/DHEA, THA/PT, 5α-THA/3a5β-THALDO, 5α-THB/11β-OH-ANDRO, THA/Cortisol, THB/Cortisol, THB/Cortisone, 6β-OH-Cortisol/11β-OH-ANDRO, THB/α-cortol, PT-ONE/Cortisone, PD/PT, PT/THS, PD/11β-OH-ETIO, 18-OH-THA/11-OXO-Et, THA/β-cortol, 17-HP/Cortisol, 5α-THB/3a5β-THALDO, THB/THF, 3a5β-THALDO/17-HP, THB/6β-OH-F, THA/6β-OH-F, α-cortolone/DHEA, THB/DHEA, 3a5β-THALDO/PT-ONE, 18-OH-THA/β-cortolone, 5α-THB/6β-OH-F, 18-OH-THA/α-cortol, 5α-THA/5-PT, 5a-THB/PT, PD/Cortisone, PD/6β-OH-F

The same samples as above will now become 165dim ratio vectors:

1.48, 1.20, 2.14, 0.19, 8.84, NaN, 29.18, NaN, NaN, NaN, 4.88, 41.88, 3.95, NaN, 3.61, 0.63, 0.91, NaN, 30.44, 0.25, NaN, 0.03, 0.66, NaN, 7.00, 0.25, NaN, NaN, NaN, 0.17, NaN, 1.74, 0.83, 0.67, 0.10, 0.24, 0.29, 0.49, 9.09, 1.09, NaN, 0.02, 0.02, 0.00, 4.25, 0.01, 0.01, NaN, 0.04, 0.01, NaN, 0.02, NaN, 0.20, 8.42, 15.60, 0.01, 0.02, NaN, NaN, NaN, 0.07, NaN, 0.26, 0.09, 0.03, 0.21, 0.09, 0.15, 0.56, 1.48, NaN, 0.64, NaN, 0.13, 0.16, NaN, 1.87, 0.43, NaN, 3.13, 0.28, NaN, 13.10, 0.01, 1.62, NaN, NaN, NaN, 0.07, 0.17, 0.30, 0.29, 0.19, 0.53, 3.30, NaN, NaN, NaN, NaN, NaN, 1.15, 15.03, 1.42, 5.67, 0.20, 0.43, 0.51, 1.36, 1.16, 1.78, 1.15, 2.55, 3.66, 4.39, 2.32, 1.19, 0.34, 0.46, NaN, 0.95, 0.93, 0.33, 0.66, 0.11, NaN, 0.36, 2.11, NaN, 0.31, 0.77, 36.62, 5.49, 0.25, 2.66, 0.37, NaN, 0.59, 2.61, 2.51, 2.04, NaN, 0.64, 0.14, 0.73, 4.74, 0.69, NaN, 0.31, 2.19, NaN, 0.10, NaN, NaN, NaN, 12.79, 1.31, NaN, NaN, NaN, NaN, 0.29, 0.65, 4.12, NaN,

1.40, 1.00, 0.24, 1.33, 28.00, 0.74, 8.00, 0.05, 1.00, 0.04, 0.40, 14.00, 0.29, 0.03, 0.24, 0.38, 0.67, 0.05, 9.00, 0.43, 191.25, 0.12, 0.43, 0.21, 0.70, 3.50, 40.26, 4.12, 54.64, 2.50, 76.50, 15.12, 0.10, 0.12, 0.02, 0.02, 0.07, 0.04, 33.00, 6.38, 0.00, 0.01, 0.01, 0.00, 138.60, 0.00, 0.01, 0.37, 0.09, 0.02, 0.03, 0.05, 0.01, 2.50, 35.00, 33.00, 0.20, 0.14, 109.29, 1530.00, 0.00, 0.01, 0.02, 0.13, 0.40, 0.02, 0.14, 0.09, 0.05, 1.55, 0.61, 0.00, 2.06, 0.45, 3.40, 1.33, 0.01, 15.57, 2.80, 0.03, 9.19, 0.81, 90.00, 17.00, 0.06, 0.13, 0.09, 72.86, 0.01, 0.01, 0.05, 2.43, 0.33, 1.67, 4.12, 0.29, 36.43, 2.21, 0.04, 0.03, 7.93, 1.76, 30.00, 12.06, 0.50, 3.50, 4.12, 3.75, 5.76, 6.36, 1.27, 1.89, 0.89, 1.56, 14.89, 0.53, 1.94, 1.57, 0.07, 0.12, 2.00, 8.75, 1.43, 3.00, 0.79, 0.24, 2.14, 1.18, 4.68, 0.21, 3.11, 77.00, 13.44, 0.27, 1.00, 2.36, 1.06, 3.33, 1.65, 1.50, 1.07, 1.81, 2.73, 0.04, 0.64, 0.50, 1.29, 95.62, 0.25, 0.85, 2.12, 0.16, 1.94, 0.79, 0.87, 4.67, 3.33, 33.00, 12.64, 1.84, 139.09, 4.38, 5.00, 0.32, 0.24,

0.40, 0.06, 0.45, 0.10, 0.09, 0.19, 0.10, 2.80, 0.65, 0.43, 0.41, 0.56, 0.06, 1.33, 0.03, 0.10, 0.59, 0.68, 0.20, 0.20, 0.00, 0.03, 0.20, 0.19, 2.39, 0.00, 0.00, 0.00, 0.00, 0.01, 0.00, 0.01, 2.61, 1.33, 3.65, 6.81, 2.25, 1.30, 0.16, 0.22, 4.26, 4.65, 2.37, 6.51, 0.36, 2.33, 4.01, 1.14, 2.17, 40.08, 2.03, 1.05, 1.55, 0.00, 0.00, 0.25, 2.35, 11.58, 0.00, 0.00, 1.52, 1.27, 26.23, 5.61, 48.31, 7.85, 2.85, 4.83, 2.80, 2.62, 12.82, NaN, 0.00, 2.44, 2.83, 1.58, 3.04, 0.45, 1.32, NaN, 0.26, 1.26, 0.00, 1.21, 0.96, 2.48, 1.42, 0.00, 2.62, 0.62, 4.27, 13.96, 22.46, 0.00, 2.56, 1.02, 0.00, 0.00, 6.96, 4.55, 0.00, 0.00, 0.00, 0.24, 0.37, 0.04, 0.01, 0.00, 0.44, 0.00, 1.22, 6.28, 0.50, 2.92, 1.12, 0.16, 0.20, 0.26, 0.23, 6.82, 6.49, 0.00, 0.00, 0.53, 9.85, 1.53, 0.00, 0.16, 0.00, 0.00, 0.49, 0.80, 2.20, 0.58, 1.02, 0.44, 0.00, 0.00, 1.05, 0.00, 0.00, 0.52, 0.00, 0.66, 0.35, 5.96, 2.27, 0.00, 1.14, 5.15, 0.00, 0.00, 0.12, 0.00, 0.50, 0.13, 0.00, 0.14, 0.00, 0.00, 0.00, 0.00, 0.00, 0.13, 0.40,

1.48, 2.06, 3.34, 0.54, 0.18, 11.93, 2.16, 2.41, 0.29, 3.36, 18.00, 7.78, 25.03, 19.47, 4.65, 6.58, 23.70, 0.88, 8.97, 1.39, 12.93, 33.63, 27.33, 145.20, 64.84, 5.97, 25.87, 1.40, 4.46, 4.03, 3.21, 48.03, 13.14, 5.44, 0.77, 0.04, 5.91, 10.06, 30.82, 1.80, 0.03, 1.69, 0.70, 0.10, 9.22, 1.29, 0.76, 523.07, 18.35, 1.19, 67.27, 3.05, 0.20, 0.06, 0.45, 2.91, 0.91, 6.23, 0.05, 0.38, 0.37, 0.89, 0.35, 6.29, 4.44, 0.37, 2.61, 2.83, 4.81, 68.27, 20.98, 0.75, 0.12,250.33, 3.39, 0.26, 0.49, 2.36, 7.09, 0.65, 4.01, 11.34, 0.10, 3.72, 2.68, 0.26, 5.31, 1.17, 0.22, 0.21, 8.34, 23.18, 9.27, 2.46, 1.35, 12.17, 0.46, 0.04, 1.63, 6.95, 0.29, 0.24, 0.89, 3.52, 90.18, 2.90, 97.73, 0.41, 4.35, 37.76,142.65, 8.16, 4.73, 17.06, 1.30, 2.41, 0.78, 8.86, 1.51, 0.13, 48.43, 0.95, 2.73, 53.31, 3.47, 1.62, 0.12, 33.70, 0.50, 2.66, 0.39, 22.18, 3.13, 2.03, 19.67, 0.37, 10.74, 6.27, 24.23, 7.46, 10.38, 0.05, 16.42, 11.60, 1.15, 43.83, 55.84, 1.03, 4.91, 31.03, 49.45, 0.68, 0.01, 60.20,195.47, 1.84, 1.96, 0.04, 0.27,138.47, 7.05, 0.21, 0.26,103.98,603.07,

1.28, 1.08, 0.98, 1.41, 15.00, 0.65, 14.88, 0.15, 1.75, 0.13, 1.27, 38.25, 1.07, 0.08, 5.25, 1.40, 3.60, 0.16, 20.75, 3.84, 5.58, 0.42, 1.95, 0.65, 3.26, 6.32, 0.58, NaN, 0.95, 4.95, 1.13, 27.42, 1.09, 0.59, 0.08, 0.08, 0.28, 34.00, 47.58, 3.31, 0.00, 0.03, 0.02, 0.00, 48.72, 0.89, 0.01, 1.66, 0.09, 0.01, 0.04, 0.09, 0.03, 0.06, 2.12, 42.75, 0.10, 0.04, 0.35, 13.25, 1.56, 0.04, 0.01, 0.25, 0.07, 0.02, 0.14, 0.07, 7.89, 0.80, 0.59, 0.13, 0.24, 0.39, 0.86, 1.31, 0.05, 0.11, 3.69, 0.82, 12.66, 0.84, 1.49, 8.88, 0.02, 0.62, 0.16, 1.25, 0.01, 0.03, 0.09, 0.39, 0.32, 0.24, 2.87, 0.99, 0.11, 0.03, 0.12, NaN, 0.10, 3.56, 31.62, 3.35, 2.76, 5.84, 18.00, 2.13, 6.13, 2.61, 3.44, 0.22, 0.37, 0.94, 0.86, 2.34, 4.82, 4.02, 0.23, 0.12, 1.66, 1.95, 2.98, 3.84, 0.36, NaN, 0.83, 3.08, 11.78, 25.78, 0.37, 12.40, 1.60, 0.66, 0.51, 1.12, NaN, 2.73, 3.64, 2.69, 2.28, 2.16, 2.84, 0.07, 0.54, 2.55, 0.90, 0.33, 1.22, 0.76, NaN, 0.24, NaN, 1.38, 1.86, 2.95, 0.78, NaN, 0.20, 1.26, 1.19, 0.14, 0.76, 1.50, 0.90,

The algorithm works with angles on the unit sphere. The samples are by nature positive since they are amounts of substance, so they are in the upper right quadrant only.

To have more room to distinguish them we normalize the data with zero mean so they spread to all four quadrants and unit variance so we can interpret the dimension weighting. This normalization is done with the training sets used in the cross-validation (so not using all the available data).

Therefore, the normalized ratio vectors used for training the algorithm look like the following:

0.20, 0.20, 0.25, -0.61, 0.13, NaN, 1.06, NaN, NaN, NaN, 0.16, 0.32, 0.22, NaN, 0.75, -0.29, -0.37, NaN, 0.04, -0.48, NaN, -0.15, -0.31, NaN, -0.20, -0.15, NaN, NaN, NaN, -0.17, NaN, -0.13, -0.23, -0.15, -0.20, -0.09, -0.19, -0.22, -0.49, -0.43, NaN, -0.25, - 0.17, -0.22, -0.52, -0.17, -0.21, NaN, -0.16, -0.17, NaN, -0.28, NaN, -0.60, -0.41, -0.10, - 0.18, -0.13, NaN, NaN, NaN, -0.28, NaN, -0.21, -0.18, -0.20, -0.18, -0.16, -0.19, -0.12, - 0.25, NaN, -0.44, NaN, -0.25, -0.41, NaN, -0.46, -0.32, NaN, -0.29, -0.41, NaN, 0.30, - 0.27, 0.29, NaN, NaN, NaN, -0.22, -0.12, -0.37, -0.21, -0.25, -0.47, -0.10, NaN, NaN, NaN, NaN, NaN, -0.21, 0.34, -0.48, -0.22, -0.51, -0.23, -0.40, -0.49, -0.33, -0.14, -0.23, - 0.24, -0.27, -0.39, -0.10, -0.44, -0.14, 0.12, NaN, -0.30, -0.46, -0.38, -0.14, -0.21, NaN, - 0.38, -0.15, NaN, -0.14, -0.38, -0.48, -0.60, -0.16, -0.28, -0.55, NaN, -0.38, -0.14, 0.02, 0.13, NaN, -0.23, -0.25, -0.13, -0.26, -0.30, NaN, -0.47, -0.12, NaN, -0.28, NaN, NaN, NaN, -0.35, -0.47, NaN, NaN, NaN, NaN, -0.69, -0.34, -0.18, NaN,

0.15, 0.09, -0.80, 0.67, 1.61, -0.19, -0.28, -0.24, -0.31, -0.41, -0.53, -0.27, -0.43, -0.25, -0.65, -0.36, -0.40, -0.27, -0.16, -0.32, 7.55, -0.15, -0.32, -0.16, -0.30, 0.03, 3.83, 0.73, 9.50, -0.10, 10.31, -0.06, -0.26, -0.17, -0.21, -0.25, -0.19, -0.23, 0.01, 1.00, -0.23, -0.25, -0.17, -0.22, 0.62, -0.17, -0.21, -0.28, -0.16, -0.17, -0.22, -0.28, -0.28, 0.19, 0.52, 0.68, -0.18, -0.13, 10.69, 10.71, -0.14, -0.35, -0.21, -0.22, -0.17, -0.20, -0.18, -0.16, -0.20, -0.12, -0.27, -0.22, -0.32, - 0.24, -0.05, -0.22, -0.13, 0.75, -0.21, -0.30, 0.93, -0.29, 6.94, 0.55, -0.27, -0.23, -0.29, 9.53, -0.19, -0.45, -0.12, -0.29, -0.21, 0.11, 0.01, -0.26, 10.12, 7.09, -0.15, -0.44, 2.26, -0.11, 1.50, 0.27, -0.39, 0.68, -0.17, 0.64, -0.28, 0.29, -0.15, -0.22, -0.36, -0.30, -0.05, -0.13, -0.34, -0.13, -0.42, -0.19, -0.29, 0.33, -0.05, -0.11, -0.13, -0.33, 1.25, -0.18, 0.22, -0.15, -0.16, -0.29, -0.46, -0.16, -0.31, 0.20, -0.52, -0.14, -0.16, -0.29, -0.22, -0.01, 0.22, -0.25, -0.15, -0.32, -0.28, 9.09, -0.48, -0.14, -0.35, -0.25, 0.07, -0.19, -0.24, -0.49, -0.32, 3.15, 9.24, -0.25, 10.74, 0.40, 0.25, -0.39, -0.19,

-0.47, -0.43, -0.69, -0.71, -0.55, -0.32, -0.78, 0.29, -0.41, -0.16, -0.53, -0.55, -0.47, -0.14, - 0.74, -0.44, -0.41, 0.02, -0.25, -0.52, -0.26, -0.15, -0.34, -0.16, -0.27, -0.16, -0.45, -0.45, -0.39, -0.18, -0.41, -0.13, -0.14, -0.13, 0.21, 4.68, -0.15, -0.20, -0.68, -0.66, 9.30, 0.10, -0.05, 0.67, -0.56, -0.13, -0.11, -0.28, -0.14, 0.73, -0.20, -0.15, 2.29, -0.66, -0.70, -0.79, -0.13, -0.08, - 0.25, -0.20, -0.03, 1.05, 8.01, 0.16, 1.54, 1.29, 0.01, -0.05, -0.12, -0.11, 0.09, NaN, -0.50, -0.22, -0.08, -0.18, 0.28, -0.59, -0.28, NaN, -0.87, -0.19, -0.46, -0.48, -0.22, 0.60, 0.10, - 0.41, 0.34, 1.99, -0.09, 0.10, 0.62, -0.30, -0.20, -0.22, -0.23, -0.40, 0.34, 0.62, -0.54, -0.38, -0.82, -0.57, -0.39, -0.57, -0.24, -0.56, -0.54, -0.46, -0.15, -0.14, -0.39, -0.28, -0.50, -0.14, - 0.56, -0.15, -0.20, 1.84, -0.23, -0.56, -0.48, -0.14, 0.97, 0.20, -0.71, -0.21, -0.31, -0.17, -0.41, -0.65, -0.65, -0.09, -0.31, -0.53, -0.69, -0.44, -0.17, -0.74, -0.61, -0.40, -0.37, -0.22, -0.23, - 0.24, -0.26, -0.40, -0.28, -0.09, -0.43, -0.33, -0.36, -0.21, -0.24, -0.57, -0.56, -0.56, -0.30, -0.29, -0.22, -0.76, -0.43, -0.40, -0.19,

0.20, 0.67, 0.91, -0.22, -0.54, 2.65, -0.65, 0.21, -0.51, 1.70, 2.21, -0.40, 3.99, 1.40, 1.18, 1.36, 1.81, 0.11, -0.16, 0.55, 0.27, 0.11, 1.72, 1.95, 0.70, 0.17, 2.30, -0.05, 0.42, - 0.05, 0.04, 0.09, 0.35, 0.02, -0.13, -0.24, -0.09, -0.03, -0.03, -0.23, -0.17, -0.13, -0.14, -0.21, -0.48, -0.15, -0.19, 2.96, 0.05, -0.14, 0.56, 0.10, 0.04, -0.64, -0.69, -0.67, -0.16, -0.10, -0.25, -0.20, -0.11, 0.62, -0.11, 0.21, -0.02, -0.13, -0.00, -0.10, -0.06, 0.36, 0.33, -0.05, -0.49, 2.44, -0.05, -0.39, -0.06, -0.42, -0.02, -0.01, -0.11, 2.05, -0.46, -0.32, -0.12, -0.19, 1.24, -0.25, - 0.15, 0.35, -0.05, 0.41, 0.13, 0.31, -0.36, 0.38, -0.10, -0.27, -0.04, 1.18, -0.44, -0.34, -0.75, -0.34, 2.49, 0.46, 1.36, -0.43, -0.35, 4.03, 0.64, -0.11, -0.08, -0.13, -0.50, -0.10, -0.49, -0.03, 1.53, -0.19, 0.30, -0.46, 0.35, 0.47, 0.19, 0.23, -0.61, 0.84, -0.25, 0.02, -0.42, -0.54, -0.64, 0.25, 0.01, -0.55, 1.04, 0.12, 0.27, 1.50, 3.13, -0.54, 3.20, 0.38, -0.01, 0.33, 0.98, -0.30, 0.59, 0.24, 1.44, 0.00, -0.38, 1.81, 2.04, -0.54, -0.42, -0.58, -0.10, 2.65, 0.33, -0.71, -0.39, 5.30, 3.48,

0.08, 0.13, -0.39, 0.76, 0.60, -0.21, 0.15, -0.22, -0.10, -0.35, -0.40, 0.25, -0.29, -0.24, 1.43, -0.08, -0.12, -0.22, -0.05, 2.75, -0.03, -0.14, -0.21, -0.16, -0.26, 0.19, -0.39, NaN, -0.21, -0.02, -0.25, -0.01, -0.21, -0.15, -0.21, -0.21, -0.19, 0.45, 0.32, 0.17, -0.22, -0.25, -0.17, -0.22, -0.15, -0.16, -0.21, -0.27, -0.16, -0.17, -0.22, -0.28, -0.25, -0.64, -0.63, 1.12, -0.18, -0.13, - 0.22, -0.11, -0.03, -0.32, -0.21, -0.21, -0.18, -0.20, -0.18, -0.16, 0.03, -0.12, -0.27, -0.19, -0.48, -0.24, -0.21, -0.23, -0.12, -0.62, -0.17, 0.08, 1.63, -0.29, -0.34, 0.02, -0.27, -0.06, -0.27, -0.24, -0.19, -0.38, -0.12, -0.36, -0.21, -0.24, -0.16, -0.22, -0.20, -0.31, -0.15, NaN, -0.51, 0.16, 1.63, -0.35, -0.32, 1.53, 0.06, 0.12, -0.26, -0.15, -0.14, -0.25, -0.40, -0.30, -0.51, -0.10, 0.02, -0.09, -0.20, -0.19, -0.29, -0.36, 0.42, -0.10, -0.18, NaN, 0.05, -0.12, 1.03, 1.60, -0.42, - 0.59, -0.66, -0.07, -0.32, -0.27, NaN, -0.19, -0.12, 0.07, 0.21, 0.10, 0.25, -0.25, -0.18, -0.29, -0.29, -0.37, -0.26, -0.14, NaN, -0.21, NaN, -0.17, -0.22, -0.52, -0.51, NaN, -0.15, -0.26, - 0.13, -0.73, -0.33, -0.32, -0.19,

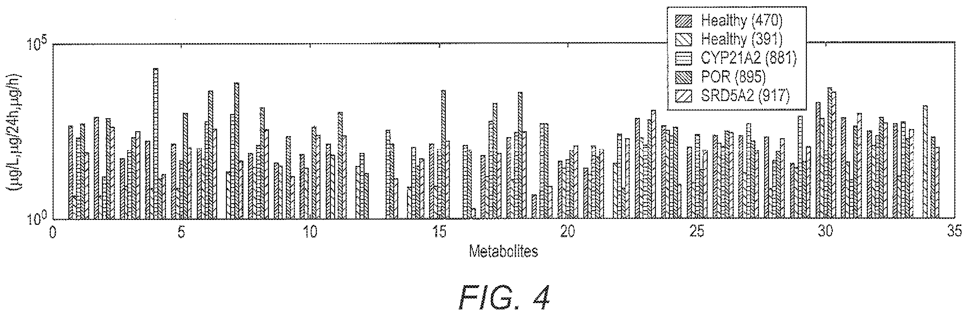

Figure 4 shows an example of an orignal 35 metabolite fingerprint.

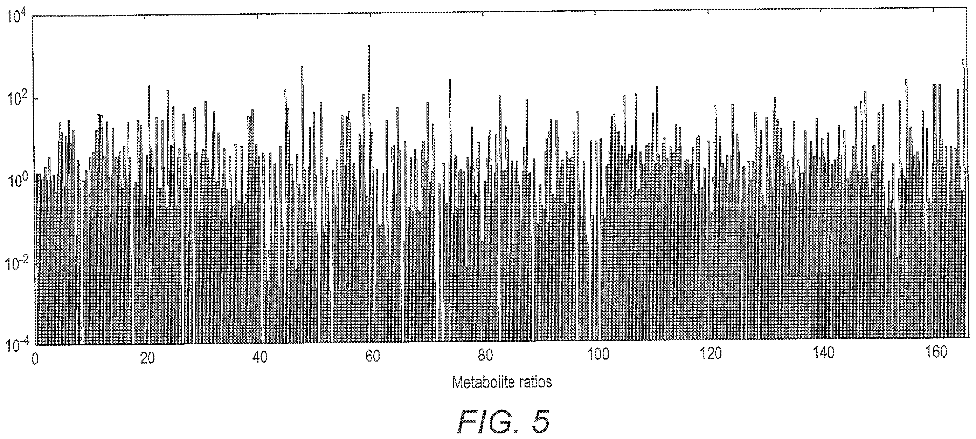

Figure 5 shows a representation of vectors for 165 dimensions using problem specific expert knowledge and ANOVA,

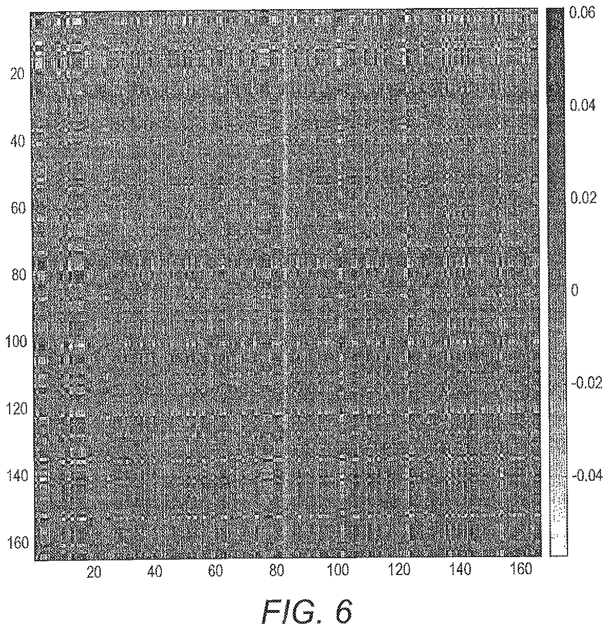

An example of the relevance matrix is visualised in Figure 6

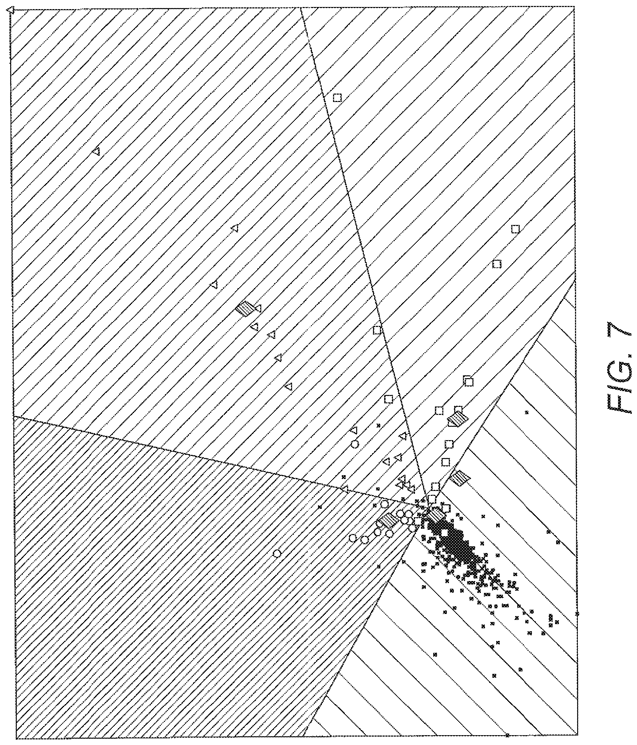

An example of a 2D angle LVQ representation is shown in Figure 7, which shows markers for different disease states compared to prototypes.

The Applicant tested the proposed techniques on the metabolomic data described above and classify the three inborn steroiodgenic conditions CYP21A2, PORD and SRD5A2 from heathly controls. Since the conditions affect enzyme activity we represent the metabolomic profiles by vectors of pair-wise steroid ratios. From the $34^2$ possible ratios they selected 165 by analysis of variance (ANOVA) of the conditions versus heathly. Furthermore, they randomly set aside over 700 healthy samples and ca. 4 samples of each condition as test set, so the majority class is down sampled. They trained the angle LVQ method using 5 fold cross-validation on the remaining data using one prototype per class and regulization with $\gamma = 0.001$. They achieved a very good mean (std) sensitivity of 0.81 (0.049) for detecting patients with one of three conditions trained, 0.73 (0.069) precision and an excellent specificity of 0.97 (0.008) for healthy controls for the relevance vector version of angle LVQ.

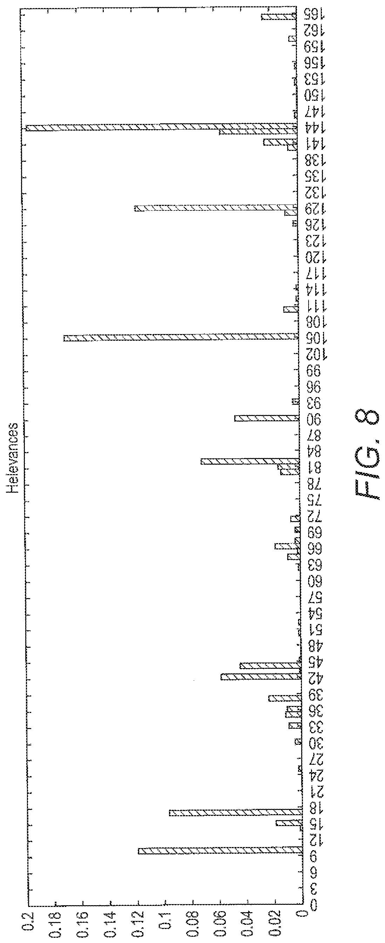

The resulting relevance vector of the best model is shown in Figure 8, where distinct steroid ratios were identified as most important for classification. Note, that even samples with 30 to 79% of its ratios missing were on average 98.7% classified correctly with this model. In direct comparison GRLVQ (using distances not angles) with mean imputation for the missing values trained on the same data splits achieves in average 0.98 (0.018) specificity and 0.81 (0.2) precision for normal profiles, but only a sensitivity of 0.42 (0.106) for patients. Increasing the complexity of the angle LVQ algorithm proposed by the applicants using a global relevance matrix could further improve sensitivity and specificity to 97% respectively.

This shows that the methodology of the presently claimed invention can be applied to complex pathways to identify a number of different disease conditions within the different pathways. This may apply to a number of different alternative pathways and to a wide range of biological systems.

Further Exemplification

The common challenges of medical datasets are 1) heterogeneous measurements, 2) missing data, and 3) imbalanced classes. In Appendix 1 a variant of Learning vector quantization (LVQ) has been introduced which is capable of handling the first 2 issues. This variant of LVQ, known as angle LVQ (ALVQ) uses cosine dissimilarity instead of Euclidean distances, a property which makes this LVQ variant robust for classification of data containing missingness. We performed the following experiments to check the performance of ALVQ in terms of its classification sensitivity, specificity, classwise accuracy, and robustness. The experiments were performed with 5 folds 5 runs cross validation. In each run of each fold the initialization of prototypes differed.

**Dataset** Urine GCMS data set with the following classes in training and test folds. The numbers mentioned in the table below are mean over 5 fold and 5 runs.

|  | Training |  | Validation |  | Generalization |
| --- | --- | --- | --- | --- | --- |
| **Healthy** | 663.2 | (664,678,677,647,650) | 165.8 | (165,151,152,182,179) | 0 |
| **CYP21A2** | 14.4 | (15,14,14,14,15) | 3.6 | (3,4,4,4,3) | 0 |
| **POR** | 16.8 | (17,16,17,17,17) | 4.2 | (4,5,4,4,4) | 17 |
| **SRD5A2** | 23.2 | (24,23,23,23,23) | 5.8 | (5,6,6,6,6) | 10 |

In the following part of the report, when referring to CYP21A2, POR, and SRD5A2 classes together, the term disease classes, and to refer to the subjects of these classes cumulatively, the term patients will be used. In the following sections performance of angle LVQ with dimension = 2; and 3, both global and local were investigated. In order to handle the missingness cost-definitions and geodesicSMOTE oversampling (appendix 1) were applied. Also, eigen-value based feature selection scheme was tried to reduce the model complexity and enable easier data interpretation.

### 1 Angle LVQ, global, 2dimensions, baseline

Angle LVQ with 2 dimensional global matrix, and exponential dissimilarity transform factor b = 1. No treatment was done on the the classifier to account for the imbalanced class data.

### 2 Angle LVQ, global, 2dimensions, with cost definitions

Angle LVQ with 2 dimensional global matrix, and exponential dissimilarity transform factor b = 1. The misclassification of patients (CYP21A2, POR or SRD5A2) to healthy was more severely penalized by the classifier.

### 3 Angle LVQ, local, 2dimensions, baseline

Angle LVQ with 2 dimensional local matrices for each of the classes (each class has its own 2_featurenb matrix), and exponential dissimilarity transform factor b = 1.

### 4 Angle LVQ, global, 3dimensions, baseline

Angle LVQ with 3 dimensional global matrix, and exponential dissimilarity transform factor b = 1. No treatment was done on the classifier to account for the imbalanced class data.

### 5 Angle LVQ, global, 3dimensions, with cost definitions

Angle LVQ with 3 dimensional global matrix, and exponential dissimilarity transform factor b = 1. The misclassification of patients (CYP21A2, POR or SRD5A2) to healthy was more severely penalized by the classifier.

### 6 Angle LVQ, global, 3dimensions, with geodesic SMOTE oversampling

Angle LVQ with 3 dimensional global matrix, and exponential dissimilarity transform factor b = 1. The classifier itself was not modified in any way but the imbalanced training set data was oversampled by a Geodesic variant of SMOTE. The oversample percent used was 400.

### 7 Angle LVQ, local, 3dimensions, baseline

Angle LVQ with 3 dimensional local matrices (each class has its own 3Xfeaturenb matrix), and exponential dissimilarity transform factor b = 1. This classifier gave more complex but classwise more precise models. In this experiment nothing was done to treat the imbalanced class issue of the dataset.

### 8 Angle LVQ, local, 3dimensions, with geodesic SMOTE oversampling

Angle LVQ with 3 dimensional local matrices, and exponential dissimilarity transform factor b = 1. In this experiment geodesic SMOTE oversampling was used to synthesize data in the minority classes in order to combat the imbalanced class issue.

### 9 Angle LVQ, local, 3dimensions, with feature selection

In this experiment tAngle LVQ with 3 dimensional local matrices, and exponential dissimilarity transform factor b = 1 was used. Using eigen value decomposition we estimated the number of features required from each class, in order to convey enough percent of varaiance of the dataset. Then, from the relevance-wise sorted features from the best model generated in section 7, the required features were selected. The following table shows the different features from different classes which were selected for each of the experimental settings S1 through S7.

In all the experiments described above, the b value in the ALVQ is 1.

Feature selection based on eigen value profile

Table 1: Number of features in each class which described a certain percentage of variance of that class. *Sometimes the same feature was among the most relevant features for more than one class.

| Settings | Healthy | CYP21A2 | POR | SRD5A2 | Total features* |
|---|---|---|---|---|---|
| | | | | | |
| S1 | 30 (97.48%) | 5 (92.61%) | 5(100%) | 5(100%) | 37 |
| S2 | 30 (97.48%) | 6 (96.82%) | 6(100%) | 6(100%) | 39 |
| S3 | 34 (98.08%) | 6 (96.82%) | 6(100%) | 6(100%) | 43 |
| S4 | 35 (98.21%) | 6 (96.82%) | 6(100%) | 6(100%) | 44 |
| S5 | 40 (98.73%) | 5 (92.61%) | 5(100%) | 5(100%) | 47 |
| S6 | 40 (98.73%) | 6 (96.82%) | 6(100%) | 6(100%) | 49 |
| S7 | 40 (98.73%) | 7 (100% ) | 7(100%) | 7(100%) | 51 |

## 10 Training on new diseases- CYP17A1 and HSD3B2

Along with new data for POR and SRD5A2 patients (the data used as generalization set in the previous experiments), data from 2 other diseases of the steroidogenic pathway was used for training and validation of angle LVQ. Based on the performance of angle LVQ for imbalanced data we selected geodesic SMOTE with 100% oversampling for countering the imbalanced class problem. The table below shows the number of subjects in each class during training and validation.

Table 2: Number of subjects in each class during training and validation in each fold

| Fold | Healthy | HSD3B2 | CYP17A1 | CYP21A2 | POR | SRD5A2 |
|---|---|---|---|---|---|---|
| Total | 829 | 22 | 28 | 18 | 38 | 39 |
| Training-1 | 652 | 18 | 23 | 15 | 31 | 32 |
| Validation-1 | 177 | 4 | 5 | 3 | 7 | 7 |
| Training-2 | 679 | 17 | 22 | 14 | 30 | 31 |
| Validation-2 | 150 | 5 | 6 | 4 | 8 | 8 |
| Training-3 | 664 | 17 | 22 | 14 | 30 | 31 |
| Validation-3 | 165 | 5 | 6 | 4 | 8 | 8 |
| Training-4 | 639 | 18 | 22 | 14 | 30 | 31 |
| Validation-4 | 191 | 4 | 6 | 4 | 8 | 8 |
| Training-5 | 683 | 18 | 23 | 15 | 31 | 31 |
| Validation-5 | 146 | 4 | 5 | 3 | 7 | 8 |

## 11 Results

11.1 Confusion matrices

In the following confusion matrices it is shown that how of the samples were correctly classified (the numbers on the diagonal) and how many were misclassified as which class (the off-diagonal). These are actually the mean confusion matrices (mean performance of 25 models from the 5 fold 5 runs cross validation in each experiment described). The numbers in parenthesis denote the variance from mean (standard deviation).

Table 3: Confusion matrices (mean and standard deviations) for Angle LVQ 2dimension and global matrices, baseline.

| validation: | | | | | | | | | |
|---|---|---|---|---|---|---|---|---|---|
| True/Pred | Healthy | | CYP21A2 | | PORD | | SRD5A2 | | Total |
| Healthy | 163.88 | (1.96) | 0.4 | (0.70) | 0.76 | (1.01) | 0.76 | (1.23) | 165.8 |
| CYP21A2 | 0 | (0) | 2.8 | (1.11) | 0.52 | (0.71) | 0.28 | (0.89) | 3.6 |
| PORD | 0.12 | (0.33) | 0.68 | (0.90) | 3.12 | (0.92) | 0.28 | (0.61) | 4.2 |
| SRD5A2 | 0.84 | (1.02) | 0.48 | (0.87) | 0.56 | (0.96) | 3.92 | (1.82) | 5.8 |
| generalization: | | | | | | | | | |
| True / Pred | Healthy | | CYP21A2 | | PORD | | SRD5A2 | | Total |
| PORD | 1.0 | (0.76) | 6.64 | (3.92) | 7.36 | (3.56) | 2.0 | (2.53) | 17 |
| SRD5A2 | 1.72 | (1.30) | 1.16 | (1.21) | 0.92 | (1.55) | 6.2 | (2.82) | 10 |

Table 4: Confusion matrices (mean and standard deviations) for Angle LVQ 2dimension and global matrices, with cost definitions.

| validation: | | | | | | | | | |
|---|---|---|---|---|---|---|---|---|---|
| True/Pred | Healthy | | CYP21A2 | | PORD | | SRD5A2 | | Total |
| Healthy | 162.28 | (2.44) | 1.04 | (1.01) | 0.92 | (1.32) | 1.56 | (1.52) | 165.8 |
| CYP21A2 | 0 | (0) | 3.04 | (1.09) | 0.52 | (1.00) | 0.04 | (0.2) | 3.6 |
| PORD | 0.12 | (0.33) | 0.88 | (0.97) | 3.12 | (1.05) | 0.08 | (0.27) | 4.2 |
| SRD5A2 | 0.89 | (0.86) | 0.72 | (1.27) | 0.68 | (0.80) | 3.60 | (1.58) | 5.8 |
| generalization: | | | | | | | | | |
| True/Pred | Healthy | | CYP21A2 | | PORD | | SRD5A2 | | Total |
| PORD | 1.28 | (0.73) | 6.4 | (3.69) | 8.4 | (3.64) | 0.92 | (1.55) | 17 |
| SRD5A2 | 1.48 | (1.12) | 0.6 | (1.63) | 1.28 | (1.74) | 6.64 | (2.84) | 10 |

Table 5: Confusion matrices (mean and standard deviations) for Angle LVQ 2dimension and local matrices, baseline.

| validation: | | | | | | | | | |
|---|---|---|---|---|---|---|---|---|---|
| True/Pred | Healthy | | CYP21A2 | | PORD | | SRD5A2 | | Total |
| Healthy | 163.2 | (2.73) | 1.04 | (1.01) | 0.92 | (1.32) | 1.56 | (1.52) | 165.8 |
| CYP21A2 | 0 | (0) | 3.04 | (1.09) | 0.52 | (1.00) | 0.04 | (0.2) | 3.6 |
| PORD | 0 | (0) | 0.88 | (0.97) | 3.12 | (1.05) | 0.08 | (0.27) | 4.2 |
| SRD5A2 | 0.28 | (0.54) | 0.72 | (1.27) | 0.68 | (0.80) | 3.60 | (1.58) | 5.8 |
| generalization: | | | | | | | | | |
| True/Pred | Healthy | | CYP21A2 | | PORD | | SRD5A2 | | Total |
| PORD | 1.24 | (0.72) | 3.12 | (2.45) | 11.68 | (2.21) | 0.96 | (1.17) | 17 |
| SRD5A2 | 1.36 | (0.63) | 0.24 | (0.43) | 0.76 | (0.52) | 7.64 | (0.75) | 10 |

Table 6: Confusion matrices (mean and standard deviations) for Angle LVQ 3 dimensions and global matrices, baseline.

| validation: | | | | | | | | | |
|---|---|---|---|---|---|---|---|---|---|
| True/Pred | Healthy | | CYP21A2 | | PORD | | SRD5A2 | | Total |
| Healthy | 164.16 | (2.23) | 0.4 | (0.57) | 0.72 | (1.2) | 0.52 | (0.91) | 165.8 |

(continued)

| validation: | | | | | | | | | |
|-------------|---------|------|---------|--------|------|--------|--------|--------|-------|
| True/Pred | Healthy | | CYP21A2 | | PORD | | SRD5A2 | | Total |
| CYP21A2 | 0 | (0) | 3.28 | (0.93) | 0.2 | (0.5) | 0.12 | (0.43) | 3.6 |
| PORD | 0 | (0) | 0.24 | (0.43) | 3.72 | (0.79) | 0.24 | (0.52) | 4.2 |
| SRD5A2 | 0.2 | (0.40) | 0.4 | (0.64) | 0.48 | (0.96) | 4.72 | (1.51) | 5.8 |
| generalization: | | | | | | | | | |
| True/Pred | Healthy | | CYP21A2 | | PORD | | SRD5A2 | | Total |
| PORD | 1.12 | (0.60) | 6.56 | (2.87) | 7.12 | (2.4) | 2.2 | (2.76) | 17 |
| SRD5A2 | 1.52 | (0.87) | 0.76 | (1.23) | 0.92 | 1.18) | 6.8 | (2.08) | 10 |

Table 7: Confusion matrices (mean and standard deviations) for Angle LVQ 3 dimension and global

| validation: | | | | | | | | | |
|-------------|---------|------|---------|--------|------|--------|--------|--------|-------|
| True/Pred | Healthy | | CVP21A2 | | PORD | | SRD5A2 | | Total |
| Healthy | 162.48 | (0.87) | 1.28 | (0.79) | 0.92 | | 1.12 | (0.88) | 165.8 |
| CVP21A2 | 0 | (0) | 3.2 | (0.76) | 0.32 | (0.47) | 0.08 | (0.27) | 3.6 |
| PORD | 0.2 | (0.4) | 0.4 | (0.50) | 3.36 | (0.86) | 0.24 | (0.52) | 4.2 |
| SRD5A2 | 0.84 | (0.74) | 0.36 | (0.56) | 0.56 | (0.82) | 4.04 | (0.84) | 5.8 |
| generalization: | | | | | | | | | |
| True/Pred | Healthy | | CVP21A2 | | PORD | | SRD5A2 | | Total |
| PORD | 1.12 | (0.72) | 7.32 | (3.67) | 6.92 | (3.27) | 1.64 | (1.91) | 17 |
| SRD5A2 | 1.24 | (0.66) | 0.28 | (0.45) | 0.28 | (0.61) | 8.2 | (1.11) | 10 |

Table 8: Confusion matrices (mean and standard deviations) for Angle LVQ 3 dimension and global matrices with geodesic oversampling.

| Validation (with 100% oversampling:) | | | | | | | | | |
|--------------------------------------|---------|--------|---------|--------|------|--------|--------|---------|-------|
| True/Pred | Healthy | | CYP21A2 | | PORD | | SRD5A2 | | Total |
| Healthy | 163.44 | (13.54) | 0.72 | (0.97) | 1 | (1.60) | 0.64 | (0.95) | 165.8 |
| CYP21A2 | 0 | (0) | 3.44 | (0.86) | 0.16 | (0.62) | 0 | (0) | 3.6 |
| PORD | 0.04 | (0.2) | 0.08 | (0.27) | 4.0 | (0.5) | 0.08 | (0.276) | 4.2 |
| SRD5A2 | 0.24 | (0.52) | 0.04 | (0.2) | 0.36 | (0.7) | 5.16 | (1.34) | 5.8 |
| generalization: | | | | | | | | | |
| True/Pred | Healthy | | CYP21A2 | | PORD | | SRD5A2 | | Total |
| PORD | 0.8 | (0.57) | 7.68 | (4.05) | 7.8 | (3.95) | 0.72 | (1.1) | 17 |
| SRD5A2 | 1.12 | (0.72) | 0.32 | (0.55) | 1 | (1.58) | 7.56 | (1.82) | 10 |
| Validation (with 400% oversampling:) | | | | | | | | | |
| True/Pred | Healthy | | CYP21A2 | | PORD | | SRD5A2 | | Total |
| Healthy | 163.28 | (13.22) | 0.72 | (0.73) | 0.96 | (1.13) | 0.84 | (0.98) | 165.8 |
| CYP21A2 | 0 | (0) | 3.4 | (0.70) | 0.04 | (0.2) | 0.16 | (0.62) | 3.6 |
| PORD | 0.04 | (0.2) | 0.08 | (0.27) | 3.92 | (0.95) | 0.16 | (0.62) | 4.2 |
| SRD5A2 | 0.16 | (0.37) | 0 | (0) | 0.16 | (0.47) | 5.48 | (0.82) | 5.8 |

(continued)

| generalization: | | | | | | | | |
|---|---|---|---|---|---|---|---|---|
| True/Pred | Healthy | | CYP21A2 | | PORD | | SRD5A2 | | Total |
| PORD | 0.88 | (0.66) | 7.4 | (3.65) | 7.4 | (4.02) | 1.32 | (2.2.1) | 17 |
| SRD5A2 | 1.16 | (0.89) | 0.36 | (0.81) | 0.64 | (0.56) | 7.84 | (1.10) | 10 |

Table 9: Confusion matrices (mean and standard deviations) for Angle LVQ 3dimension and local matrices baseline.

| validation: | | | | | | | | | |
|---|---|---|---|---|---|---|---|---|---|
| True/Pred | Healthy | | CYP21A2 | | PORD | | SRD5A2 | | Total |
| Healthy | 163.48 | (2.36) | 0.68 | (0.8) | 0.8 | (1.15) | 0.84 | (1.14) | 165.8 |
| CYP21A2 | 0 | (0) | 3.56 | (0.58) | 0.04 | (0.2) | 0 | (0) | 3.6 |
| PORD | 0 | (0) | 0.16 | (0.37) | 4.04 | (0.61) | 0 | (0) | 4.2 |
| SRD5A2 | 0.28 | (0.54) | 0 | (0) | 0.08 | (0.27) | 5.44 | (0.76) | 5.8 |
| generalization: | | | | | | | | | |
| True/Pred | Healthy | | CYP21A2 | | PORD | | SRD5A2 | | Total |
| PORD | 1.4 | (0.64) | 3.52 | (2.14) | 11.72 | (2.4) | 0.36 | (0.81) | 17 |
| SRD5A2 | 1.52 | (0.91) | 0 | (0) | 0.88 | (0.33) | 7.6 | (0.91) | 10 |

Table 10: Confusion matrices (mean and standard deviations) for Angle LVQ 3dimension and local matrices with geodesic oversampling.

| validation with oversampling-100%: | | | | | | | | | |
|---|---|---|---|---|---|---|---|---|---|
| True/Pred | Healthy | | CYP21A2 | | PORD | | SRD5A2 | | Total |
| Healthy | 164.08 | (11.15) | 0.48 | (0.71) | 0.4 | (0.64) | 0.84 | (0.98) | 165.8 |
| CYP21A2 | 0 | (0) | 3.56 | (0.50) | 0.04 | (0.2) | 0 | (0) | 3.6 |
| PORD | 0 | (0) | 0.16 | (0.37) | 4.04 | (0.35) | 0 | (0) | 4.2 |
| SRD5A2 | 0.28 | (0.45) | 0.04 | (0.2) | 0.04 | (0.2) | 5.44 | (0.71) | 5.8 |
| generalization: | | | | | | | | | |
| True/Pred | Healthy | | CVP21A2 | | PORD | | SRD5A2 | | Total |
| PORD | 1.28 | (0.61) | 3.24 | (1.98) | 12.28 | (2.15) | 0.2 | (0.5) | 17 |
| SRD5A2 | 1.56 | (1.0) | 0 | (0) | 0.88 | (0.33) | 7.56 | (1.0) | 10 |
| Validation with oversampling=400%: | | | | | | | | | |
| True/Pred | Healthy | | CVP21A2 | | PORD | | SRD5A2 | | Total |
| Healthy | 163.8 | (13.41) | 0.56 | (0.76) | 0.8 | (1.22) | 0.64 | (0.7) | 165.8 |
| CVP21A2 | 0 | (0) | 3.48 | (0.58) | 0.08 | (0.4) | 0.04 | (0.2) | 3.6 |
| PORD | 0.04 | (0.2) | 0.08 | (0.27) | 4.08 | (0.49) | 0 | (0) | 4.2 |
| SRD5A2 | 0.12 | (0.33) | 0 | (0) | 0 | (0) | 5.68 | (0.55) | 5.8 |
| generalization: | | | | | | | | | |
| True/Pred | Healthy | | CYP21A2 | | PORD | | SRD5A2 | | Total |
| PORD | 1.28 | (0.67) | 2.68 | (1.1) | 12.84 | (1.28) | 0.2 | (0.48) | 17 |
| SRD5A2 | 1.68 | (0.8) | 0.04 | (0.2) | 0.92 | (0.27) | 7.36 | (0.75) | 10 |

The following table represents the performance of the angle LVQ classifier on the new diseases and updated GCMS dataset.

Table 12 Confusion matrices from global and local angle LVQ classifier trained for 6-**class problem.**

| Angle LVQ local matrix | | | | | | | | | | | | | |
|---|---|---|---|---|---|---|---|---|---|---|---|---|---|
| **True/Pred** | **Healthy** | | **HSD3B2** | | **CYP17A1** | | **CYP21A2** | | **PORD** | | **SRD5A2** | | **Total** |
| **Healthy** | 161.84 | (22.83) | 0.60 | (2.7) | 0.36 | (1.4) | 1.64 | | 0.52 | (0.77) | 0.84 | (2.8) | 165.8 |
| **HSD3B2** | 0.96 | (0.53) | 3.16 | (0.89) | 0.08 | (0.27) | 0 | (0) | 0.04 | (0.2) | 0.16 | (0.47) | 4.4 |
| **CYP17A1** | 0.12 | (0.33) | 0.16 | (0.62) | 4.92 | (1.18) | 0.08 | (0.4) | 0.28 | (0.45) | 0.04 | (0.2) | 5.6 |
| **CYP21A2** | 0 | (0) | 0.04 | (0.2) | 0.04 | (0.2) | 3.04 | (0.84) | 0.40 | (0.5) | 0.08 | (0.27) | 3.6 |
| **POR** | 0.24 | (0.52) | 0 | (0) | 0.04 | (0.2) | 0.24 | (0.59) | 6.68 | (1.21) | 0.40 | (0.5) | 7.6 |
| **SRD5A2** | 0.36 | (0.56) | 0.08 | (0.27) | 0.16 | (0.37) | 0 | (0) | 0.04 | (0.2) | 7.16 | (0.89) | 7.8 |
| Angle LVQ global matrix | | | | | | | | | | | | | |
| **True/Pred** | **Healthy** | | **HSD3B2** | | **CYP17A1** | | **CYP21A2** | | **PORD** | | **SRD5A2** | | **Total** |
| **Healthy** | 161.08 | (21.38) | 0.64 | (2.19) | 1.28 | (5.38) | 0.68 | (2.39) | 1.36 | (4.14) | 0.76 | (1.01) | 165.8 |
| **HSD3B2** | 0.56 | (0.65) | 3.44 | (1.0) | 0.08 | (0.27) | 0.16 | (0.37) | 0.04 | (0.2) | 0.12 | (0.43) | 4.4 |
| **CYP17A1** | 0.20 | (0.5) | 0.08 | (0.27) | 4.80 | (0.86) | 0.04 | (0.2) | 0.40 | (0.57) | 0.08 | (0.27) | 5.6 |
| **CYP21A2** | 0.04 | (0.2) | 0.04 | (0.2) | 0.04 | (0.2) | 3.32 | (0.74) | 0.12 | (0.33) | 0.04 | (0.2) | 3.6 |
| **POR** | 0.20 | (0.5) | 0.16 | (0.47) | 0.08 | (0.27) | 0.52 | (0.82) | 6.20 | (1.55) | 0.44 | (0.50) | 7.6 |
| **SRD5A2** | 0.56 | (1.12) | 0.12 | (0.33) | 0.20 | (0.5) | 0.16 | (0.37) | 0.32 | (1.02) | 6.44 | (2.25) | 7.8 |

The big variances are due to 2 over-simplified models (so the training performance is equally bad). The other 23 models in each of the cases (global and local) work very well (with almost only the diagonal elements filled in their respective confusion matrices).

**11.2 Bar plot representation of performance of reduced models**

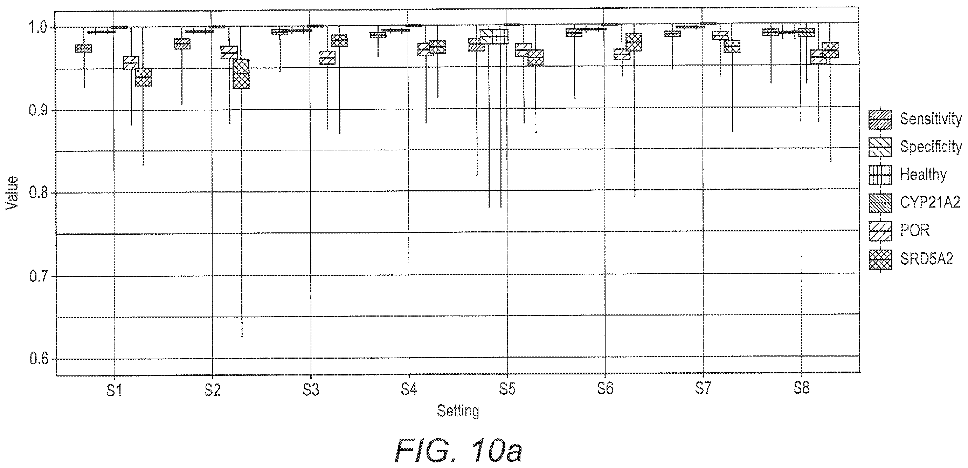

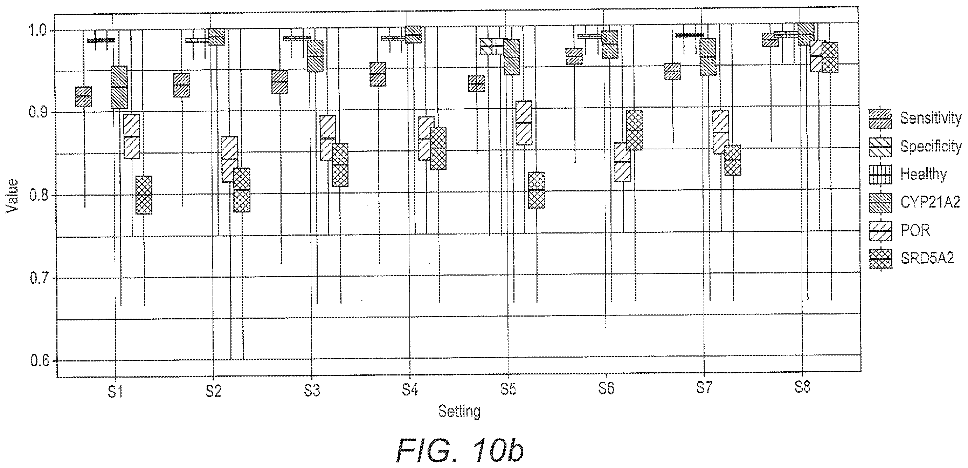

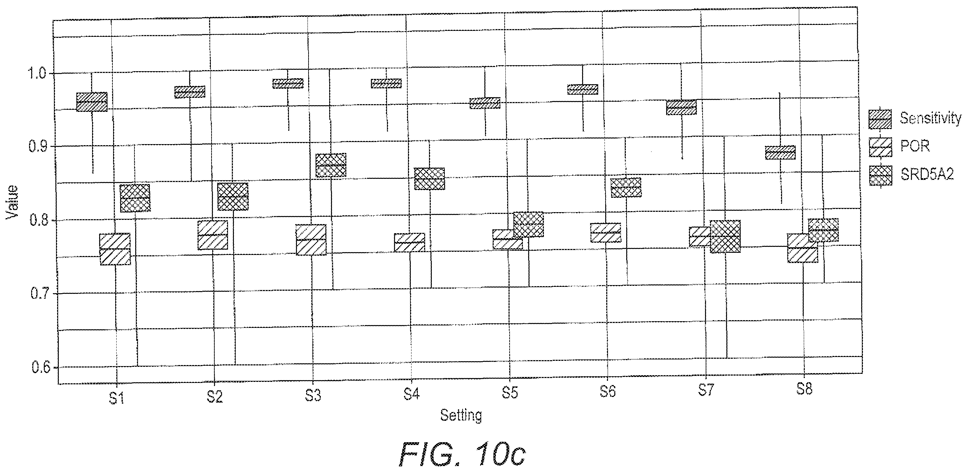

The sensitivity, specificity, classwise accuracy of healthy and each of the disease classes from validation set, and sensitivity, and classwise accuracy of POR and SRD samples forming the generalization set was plotted in the form of bar graphs (Figure 10).

The baseline setting is the local ALVQ model with full feature set, and without any strategy to handle imbalanced classes. The validation set sensitivity, specificity, classwise accuracy of Healthy, CYP21, POR, and SRD5A2 for the mentioned settings are given below:

The fact that reduction of complexity by feature selection does not adversely affect the performance of the angle LVQ classifier, shows that this is robust.

| | | | Validation set | | | |
|---|---|---|---|---|---|---|
| Settings | Sensitivity | Specificity | accuracy (Healthy) | accuracy (CYP21A2) | accuracy (POR) | accuracy (SRD5A2) |
| S1 | 0.91 (0.058) | 0.98 (0) | 0.98 (0) | 0.93 (0.11) | 0.83 (0.18) | 0.77 (0.13) |
| S2 | 0.93 (0.07) | 0.98 (0.01) | 0.98 (0.01) | 0.99 (0.02) | 0.81 (0.16) | 0.76 (0.16) |
| S3 | 0.93 (0.06) | 0.98 (0.01) | 0.98 (0.01) | 0.94 (0.06) | 0.82 (0.17) | 0.81 (0.15) |
| S4 | 0.94 (0.06) | 0.98 (0.01) | 0.98 (0.01) | 0.99 (0.02) | 0.85 (0.15) | 0.83 (0.13) |
| S5 | 0.93 (0.05) | 0.97 (0.02) | 0.97 (0.02) | 0.93 (0.14) | 0.85 (0.15) | 0.78 (0.11) |
| S6 | 0.96 (0.04) | 0.98 (0.01) | 0.98 (0.01) | 0.97 (0.05) | 0.83 (0.12) | 0.87 (0.11) |
| S7 | 0.94 (0.05) | 0.98 (0) | 0.98 (0) | 0.96 (0.08) | 0.85 (0.14) | 0.83 (0.08) |
| baseline | 0.98 (0.01) | 0.98 (0.01) | 0.98 (0.01) | 0.98 (0.02) | 0.96 (0.08) | 0.94 (0.1) |

**Table 12: Performance on the validation set**

| | | Generalization set | |
|---|---|---|---|
| Settings | Sensitivity | accuracy (POR) | accuracy (SRD5A2) |
| S1 | 0.96 (0.04) | 0.73 (0.12) | 0.83 (0.09) |
| S2 | 0.97 (0.04) | 0.76 (0.09) | 0.82 (0.09) |
| S3 | 0.98 (0.03) | 0.73 (0.13) | 0.86 (0.07) |
| S4 | 0.97 (0.02) | 0.75 (0.06) | 0.84 (0.05) |
| S5 | 0.95 (0.03) | 0.71 (0.11) | 0.78 (0.08) |
| S6 | 0.96 (0.03) | 0.76 (0.06) | 0.83 (0.06) |
| S7 | 0.94 (0.04) | 0.74 (0.07) | 0.76 (0.11) |
| baseline | 0.87 (0.03) | 0.69 (0.13) | 0.76 (0.08) |

**Table 13: Performance on the generalization set**

**11.3 Data distribution in 2D and 3D projections**

This subsection contains the classification of the dataset by angle LVQ with dimension 2 and 3.

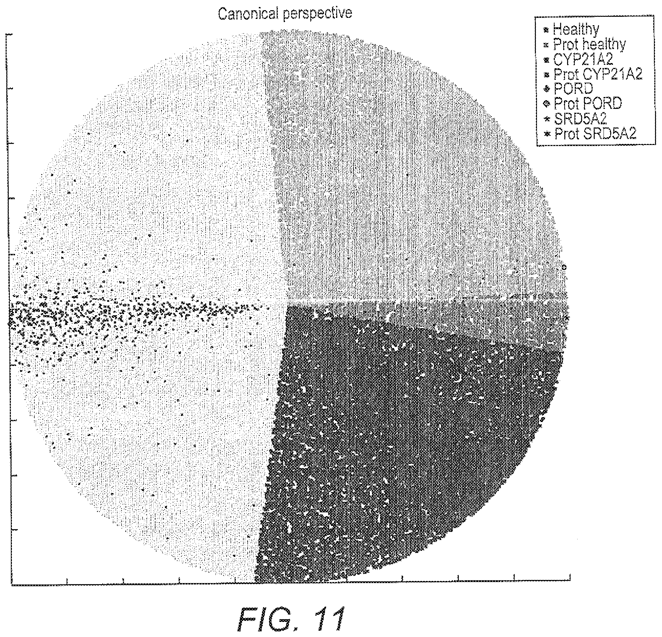

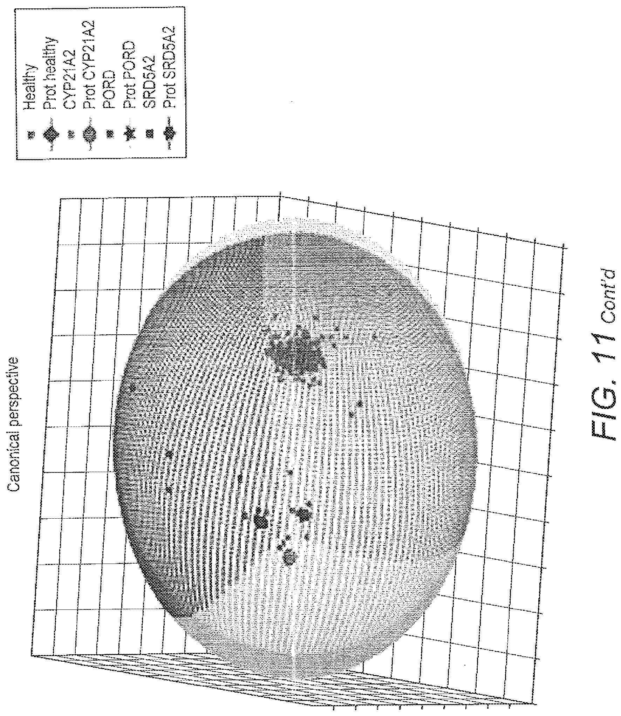

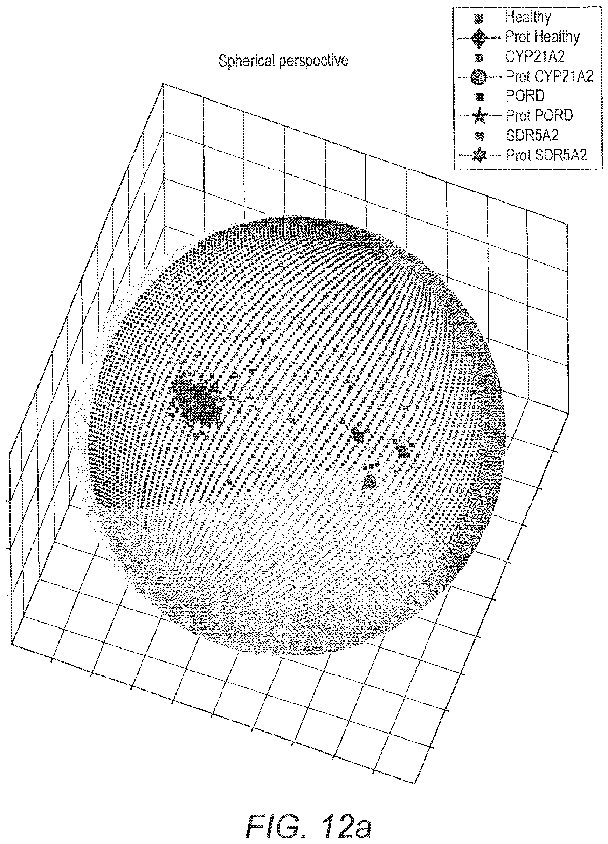

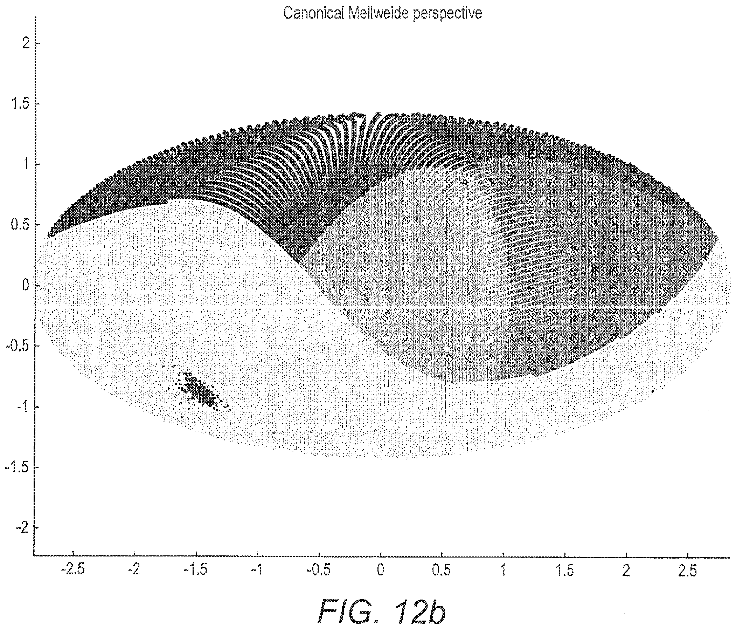

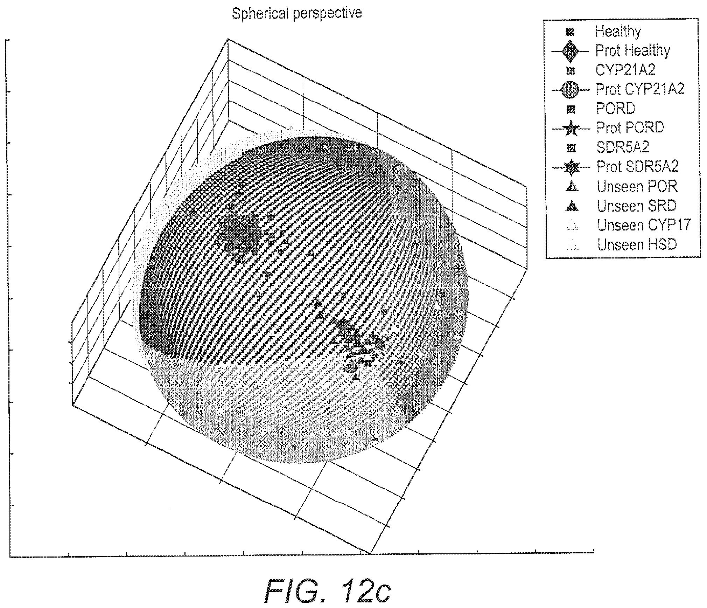

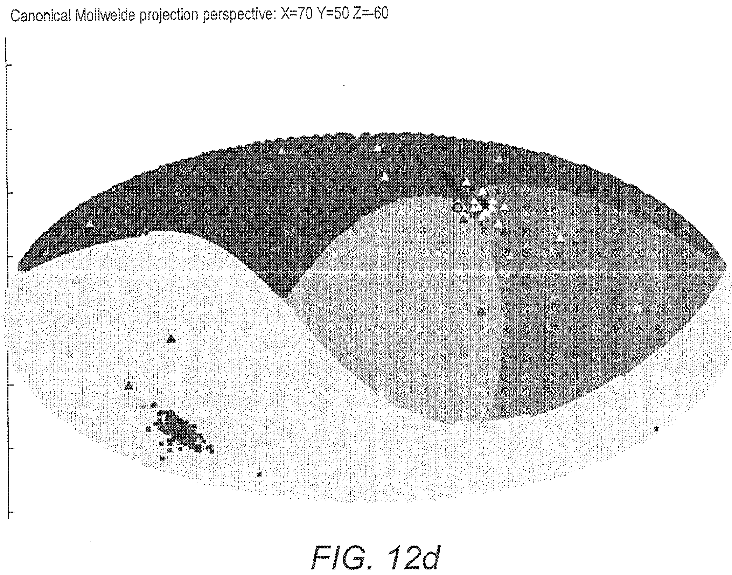

The ALVQ classifier with higher dimension not only does better classification but also gives a nice visualization of the data as classified by it (see Figure 11). From our experiments we found that ALVQ with dimension 3 performed better than ALVQ with dimension 2. Hence for the following part we investigated this higher dimension of ALVQ in detail. Also all experiments unless otherwise mentioned, were performed with ALVQ with *dimension* = 3. In Figure 12 the 3 dimensional sphere and its Mollweide projection are shown. These figures also contain the result of application of the classifier trained on only the disease classes CYP21A2, POR and SRD5A2, to classify unseen samples from POR and SRD5A2, and totally new disease data,- HSD3B2 and CYP17A1.

**11.4 Projection of classified data on the sphere and its corresponding map-projection**

The first 2 sub-figures of figure 12 shows the data classified by 3 dimension global angle LVQ and projected on a sphere. Then we used this 4-class classifier to predict the class of the new (and unseen) data from diseases POR, SRD5A2, HSD3B2 and CYP17A1. Our aim here was to see where the classifier which has no knowledge about the new diseases (HSD3B2 and CYP17A1) would place them on the sphere. Next we trained our classifier for the 6-class problem. In the following figures we show the data from 6 classes classified by the angle LVQ classifier.

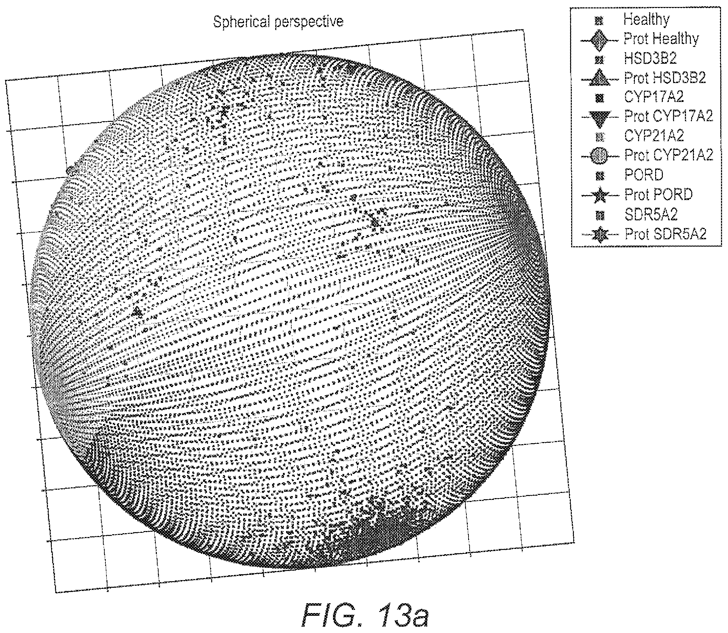

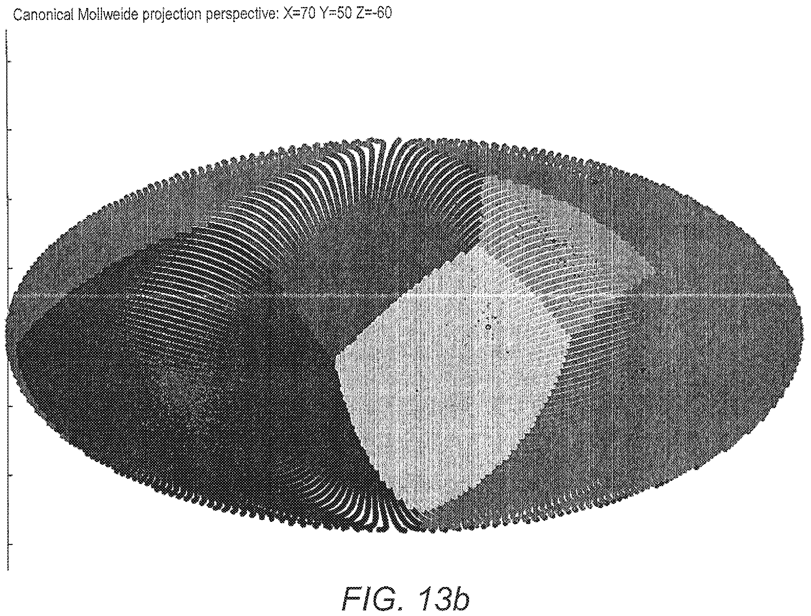

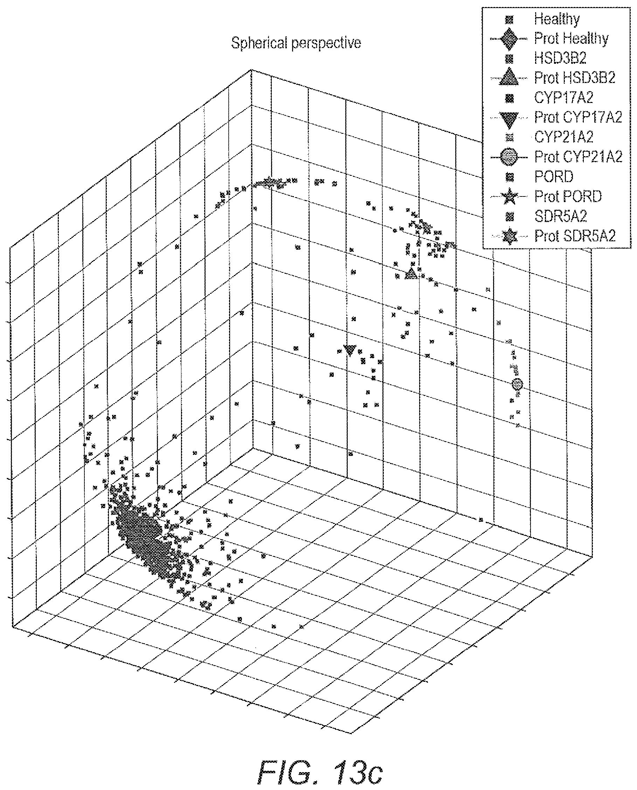

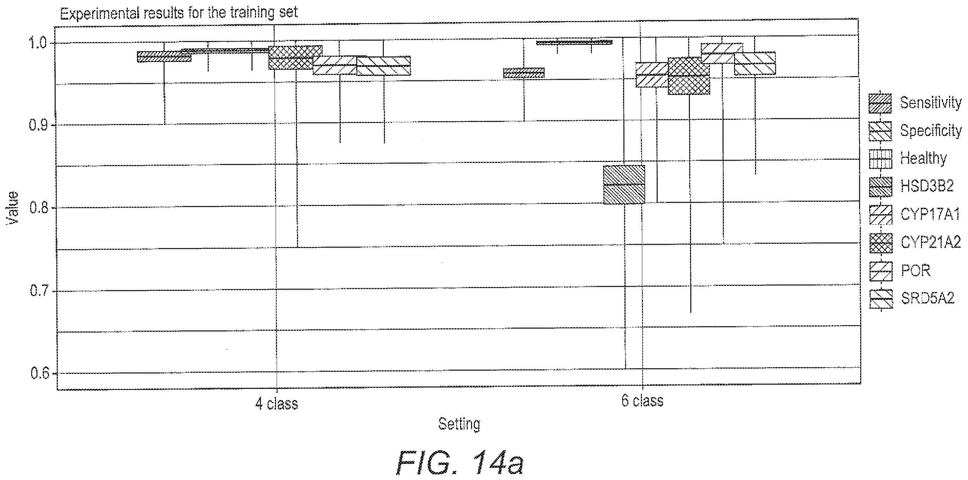

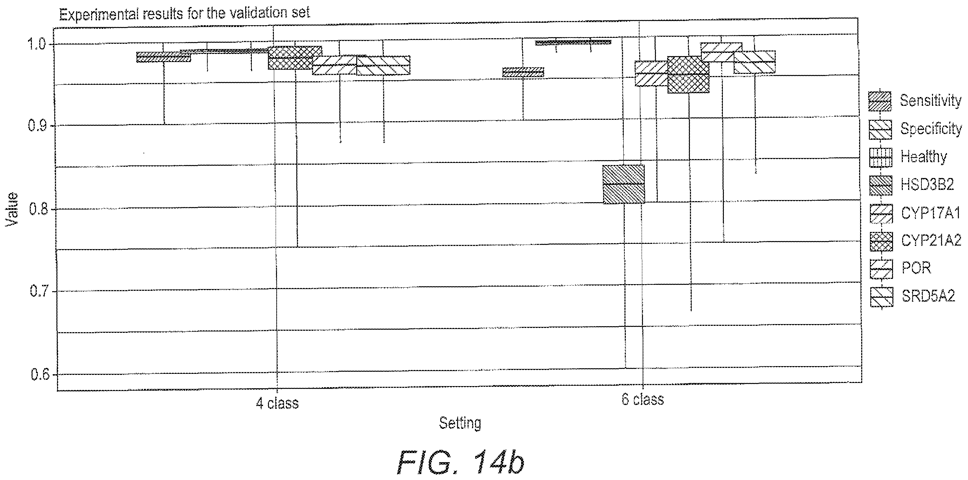

From Figure 13 it can be seen that angle LVQ coupled with geodesic SMOTE oversampling can handle imbalanced classes and can do 6-class classification with quite good class-wise accuracy (table 11). Figure 14 compares the performance of the ALVQ 3 dimension classifier with local matrices, for 4 class problem and 6 class problem.

**12 Discussion and conclusion**

The boxplots Figure 12 and the confusion matrices from tab 11 show that the disease HSD3B2 is more difficult to identify than other diseases in the dataset we investigated. Despite that, the results from tab 11, figure 13, and figure 14 indicates that ALVQ with 3 dimensions, both global (with cost-definitions to adjust for the imbalanced classes) and local, performs very well even for 6-class problem with imbalanced classes.

Tables 14 and 13, and figure 10 indicate that overfitting can be taken care of by reducing the complexity of the model by reducing number of features but without having to compromise with the classifier performance.

**References**

[1] Kerston Bunte, Petra Schneider, Barbara Hammer, Frank-Michael Schleif, Thomas Villmann, and Michael Bichl. Limited Rank Matrix Learning - Discriminative Dimension Reduction and Visualization. Neural Networks, 26(4):159-173, February 2012.

[2] Barbara Hammer, Marc Strickert, and Thomas Villmann. On the generalization ability of grlvq networks. Neural Processing Letters, 21(2):109-120, 2005.

[3] Barbara Hammer, and Thomas Villmann. Generalized relevance learning vector quantization. Neural Networks, 15(8-9):1059 - 1068, 2002.

[4] A.S. Sato and K. Yamada. Generalized learning vector quantization. In Advances in Neural Information Processing Systems, volume 8, pages 423-429, 1996.

[5] P, Schneider, M, Michl and B. Hammer. Relevance matrices in learning vector quantization. In M. Verleysen, editor, Proc. of the 15th European Symposium on Artificial Neural Networks (ESANN), pages 37-43, Bruges, Belgium, 2007. D-side publishing.

[6] Nitesh V. et al. J. Artificial Intelligence Research 16:321-357, 2002.

[7] P.T. Fletcher et al. IEEE Trans. On Medical Imaging 23(8):995-1005, 2004

[8] R.C. Wilson et al. IEEE Trans. Pattern Anal. Mach. Intell 36(11) 2255-2269, 2014

**Claims**

1.  A computer program for identifying a disease state, the computer program when executed on a processor configured to perform the steps of:

    (i) collecting metabolic data from a plurality of subjects, the metabolic data including an amount of pregnenolone and an amount of metabolites of pregnenolone for each subject, and/or including an amount of 11-deoxycortisol and an amount of metabolites of 11-deoxycortisol for each subject;

(ii) presenting the data as vectors with dimensions corresponding to different biomarkers, wherein the different biomarkers include a precursor biomarker corresponding to pregnenolone and a metabolite biomarker corresponding to a metabolite of pregnenolone, and/or a precursor biomarker corresponding to 11-deoxycortisol and a metabolite biomarker corresponding to a metabolite of 11-deoxycortisol;

(iii) training a prototype vector for a disease state of an inborn steroidogenic disorder by weighting the importance of either individual dimensions, or the interplay among multiple dimensions when calculating angles of the vectors, such that there is a minimum variation of angle within a disease class and/or a maximum variation of angle compared to a different disease class and wherein the training uses Angle Learning Vector Quantization (ALVQ); and

(iv) comparing the trained prototype vector to two or more angles of vectors of metabolic data from a patient to detect the presence of, or follow the progression of, the disease state in that patient.

2. A computer program according to claim 1, wherein the vectors are weighted by at least one relevance matrix.

3. A computer program according to any preceding claim, wherein a vector of a precursor biomarker is compared to a vector of a metabolite of the precursor biomarker.

4. A computer program according to any preceding claim, wherein the biomarkers are detected by mass spectrometry.

5. A computer program according to any preceding claim further comprising the step of identifying a plurality of biomarkers diagnostic of the disease.

6. A computer program for detecting a disease state, following progression of a disease state or providing a fingerprint of a disease state, the computer program configured to collect metabolic data and perform the method according to any preceding claim and transmitting information to a use of the disease state or fingerprint.

7. A computer readable medium storing instructions which when performed carry out the steps of any of claims 1 to 5.

8. An electronic device having a processor and a memory, the memory storing instructions which when carried out cause the processor to carry out the steps of claims 1 to 5 and transmit information regarding the disease state or fingerprint to a user.

**Patentansprüche**

1. Computerprogramm zum Identifizieren eines Krankheitszustandes, wobei das Computerprogramm, wenn es auf einem Prozessor ausgeführt wird, konfiguriert ist, die folgenden Schritte durchzuführen:

(i) Sammeln von Stoffwechseldaten von einer Vielzahl von Subjekten, wobei die Stoffwechseldaten eine Menge an Pregnenolon und eine Menge an Metaboliten von Pregnenolon für jedes Subjekt einschließen und/oder eine Menge an 11-Desoxycortisol und eine Menge an Metaboliten von 11-Desoxycortisol für jedes Subjekt einschließen;

(ii) Darstellen der Daten als Vektoren mit Dimensionen, die verschiedenen Biomarkern entsprechen, wobei die verschiedenen Biomarker einen Vorläuferbiomarker, der Pregnenolon entspricht, und einen Metabolitenbiomarker, der einem Metaboliten von Pregnenolon entspricht, und/oder einen Vorläuferbiomarker, der 11-Desoxycortisol entspricht, und einen Metabolitenbiomarker, der einem Metaboliten von 11-Desoxycortisol entspricht, einschließen;

(iii) Trainieren eines Prototypvektors für einen Krankheitszustand einer angeborenen steroidogenen Störung durch Gewichten der Wichtigkeit entweder einzelner Dimensionen oder des Zusammenspiels zwischen mehreren Dimensionen bei der Berechnung von Winkeln der Vektoren, sodass es eine minimale Variation des Winkels innerhalb einer Krankheitsklasse und/oder eine maximale Variation des Winkels im Vergleich zu einer anderen Krankheitsklasse gibt und wobei das Trainieren Winkel-Lern-Vektor-Quantisierung (ALVQ) verwendet; und

(iv) Vergleichen des trainierten Prototypvektors mit zwei oder mehr Winkelvektoren von Stoffwechseldaten eines Patienten, um das Vorhandensein des Krankheitszustands bei diesem Patienten zu erkennen oder dessen Fortschreiten zu verfolgen.

2. Computerprogramm nach Anspruch 1, wobei die Vektoren durch mindestens eine Relevanzmatrix gewichtet werden.

**3.** Computerprogramm nach einem der vorstehenden Ansprüche, wobei ein Vektor eines Vorläuferbiomarkers mit einem Vektor eines Metaboliten des Vorläuferbiomarkers verglichen wird.

**4.** Computerprogramm nach einem der vorstehenden Ansprüche, wobei die Biomarker durch Massenspektrometrie nachgewiesen werden.

**5.** Computerprogramm nach einem der vorstehenden Ansprüche, ferner umfassend den Schritt zum Identifizieren einer Vielzahl von Biomarkern, die für die Krankheit diagnostisch sind.

**6.** Computerprogramm zum Erkennen eines Krankheitszustands, zum Verfolgen des Fortschreitens eines Krankheitszustands oder zum Bereitstellen eines Fingerabdrucks eines Krankheitszustands, wobei das Computerprogramm konfiguriert ist, um Stoffwechseldaten zu sammeln und das Verfahren nach einem der vorstehenden Ansprüche durchzuführen und Informationen über die Verwendung des Krankheitszustands oder des Fingerabdrucks zu übertragen.

**7.** Computerlesbares Medium, das Anweisungen speichert, die, wenn sie durchgeführt werden, die Schritte nach einem der Ansprüche 1 bis 5 ausführen.

**8.** Elektronische Vorrichtung mit einem Prozessor und einem Speicher, wobei der Speicher Anweisungen speichert, die, wenn sie ausgeführt werden, den Prozessor veranlassen, die Schritte der Ansprüche 1 bis 5 auszuführen und Informationen über den Krankheitszustand oder den Fingerabdruck an einen Benutzer zu übertragen.


**Revendications**

**1.** Programme informatique permettant d'identifier un état pathologique, le programme informatique, lorsqu'il est exécuté sur un processeur, étant configuré pour réaliser les étapes suivantes :

(i) la collecte de données métaboliques auprès d'une pluralité de sujets, les données métaboliques comportant une quantité de prégnénolone et une quantité de métabolites de la prégnénolone pour chaque sujet, et/ou comportant une quantité de 11-désoxycortisol et une quantité de métabolites du 11-désoxycortisol pour chaque sujet ;
(ii) la présentation des données sous forme de vecteurs dont les dimensions correspondent à différents biomarqueurs, dans lequel les différents biomarqueurs comportent un biomarqueur précurseur correspondant à la prégnénolone et un biomarqueur métabolite correspondant à un métabolite de la prégnénolone, et/ou un biomarqueur précurseur correspondant au 11-désoxycortisol et un biomarqueur métabolite correspondant à un métabolite du 11-désoxycortisol ;
(iii) l'entraînement d'un vecteur prototype pour un état pathologique d'un trouble stéroïdogène congénital en pondérant l'importance soit de dimensions individuelles, soit de l'interaction entre plusieurs dimensions lors du calcul d'angles des vecteurs, de telle sorte qu'il existe une variation minimale d'angle au sein d'une classe de maladie et/ou une variation maximale d'angle par rapport à une autre classe de maladie, et dans lequel l'entraînement utilise la quantification vectorielle d'apprentissage d'angle (ALVQ) ; et
(iv) la comparaison du vecteur prototype entraîné à deux angles ou plus de vecteurs de données métaboliques provenant d'un patient pour détecter la présence de l'état pathologique ou d'en suivre la progression chez ce patient.

**2.** Programme informatique selon la revendication 1, dans lequel les vecteurs sont pondérés par au moins une matrice de pertinence.

**3.** Programme informatique selon une quelconque revendication précédente, dans lequel un vecteur d'un biomarqueur précurseur est comparé à un vecteur d'un métabolite du biomarqueur précurseur.

**4.** Programme informatique selon une quelconque revendication précédente, dans lequel les biomarqueurs sont détectés par spectrométrie de masse.

**5.** Programme informatique selon une quelconque revendication précédente, comprenant en outre l'étape suivante : l'identification d'une pluralité de biomarqueurs diagnostiques de la maladie.

**6.** Programme informatique permettant de détecter un état pathologique, suivre la progression d'un état pathologique ou fournir une empreinte d'un état pathologique, le programme informatique étant configuré pour collecter des données métaboliques et réaliser le procédé selon une quelconque revendication précédente et transmettre des informations à un utilisateur concernant l'état pathologique ou l'empreinte.

**7.** Support lisible par ordinateur stockant des instructions qui, lorsqu'elles sont réalisées, mettent en œuvre les étapes selon l'une quelconque des revendications 1 à 5.

**8.** Dispositif électronique comportant un processeur et une mémoire, la mémoire stockant des instructions qui, lorsqu'elles sont mises en œuvre, amènent le processeur à mettre en œuvre les étapes selon les revendications 1 à 5 et à transmettre des informations concernant l'état pathologique ou l'empreinte à un utilisateur.

FIG. 1

Adrenal Steroidogenesis

Mineralocorticoid precursors

Deoxycortico-sterone → (CYP11B2 / CYP11B1) → Cortico-sterone → (CYP11B2) →

11-Deoxycortisol → (CYP11B1) → Cortisol

Testosterone → (SRD5A2) → DHT

Androgens -SEX

*FIG. 1 Cont'd*

27

Mineralocorticoids
-SALT

18OH-Cortico-
sterone

CYP11B2

Aldosterone

HSD11B2

HSD11B1

Cortisone

Glucocorticoids-
SUGAR

*FIG. 1* Cont'd

Healthy control (n=88)

Androgens and androgen precursor

Mineralocorticoids and MC precurcors

Glucocorticoid precursors

Gluco-corticoids

Urinary steroid excretion (μg/24hr)

An 1 · Etio 2 · DHEA 3 · 16α-DHEA 4 · 5-PT 5 · 5-PD 6 · THA 7 · 5α-THA 8 · THB 9 · 5α-THB 10 · 5α5β-THAldo 11 · THDOC 12 · 5α-THDOC 13 · PD 14 · 3α5α-17HP 15 · 17-HP 16 · PT 17 · P'TONE 18 · THS 19 · F 20 · 6β-OH-cortisol 21 · THF 22 · 5α-THF 23 · α-cortol 24 · β-cortol 25 · 11β-OH-Andro 26 · 11β-OH-Etio 27 · E 28 · THE 29 · α-cortolone 30 · β-cortolone 31 · 11-Oxo-Etio 32

*FIG. 2*

29

*FIG. 3*

*FIG. 4*

*FIG. 5*

*FIG. 6*

FIG. 7

*FIG. 8*

FIG. 9

FIG. 10a

FIG. 10b

FIG. 10c

Canonical perspective

FIG. 11

Canonical perspective

FIG. 11 Cont'd

Legend:
- Healthy
- Prot healthy
- CYP21A2
- Prot CYP21A2
- PORD
- Prot PORD
- SRD5A2
- Prot SRD5A2

FIG. 12a

Canonical Mellweide perspective

*FIG. 12b*

Spherical perspective

Legend:
- ▦ Healthy
- ◆ Prot Healthy
- ▦ CYP21A2
- ● Prot CYP21A2
- ▦ PORD
- ✦ Prot PORD
- ▦ SDR5A2
- ✳ Prot SDR5A2
- ▲ Unseen POR
- ▲ Unseen SRD
- ▲ Unseen CYP17
- ▲ Unseen HSD

*FIG. 12c*

EP 3 510 508 B1

Canonical Mollweide projection perspective: X=70 Y=50 Z=-60

FIG. 12d

FIG. 13a

Canonical Mollweide projection perspective: X=70 Y=50 Z=-60

FIG. 13b

Spherical perspective

FIG. 13c

Experimental results for the training set

FIG. 14a

Experimental results for the validation set

Legend: Sensitivity, Specificity, Healthy, HSD3B2, CYP17A1, CYP21A2, POR, SRD5A2

Setting: 4 class, 6 class

Value axis: 0.6, 0.7, 0.8, 0.9, 1.0

*FIG. 14b*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010092363 A
- US 20140220580 A1
- US 20120040332 A1

**Non-patent literature cited in the description**

- **WIEBKE ARLT et al.** Urine Steroid Metabolomics as a Biomarker Tool for Detecting Malignancy in Adrenal Tumors. *Journal of clinical Endocrinology & Metabolism*, 01 December 2011, vol. 96 (12), 3775-3784
- **KERSTON BUNTE** ; **PETRA SCHNEIDER** ; **BARBARA HAMMER** ; **FRANK-MICHAEL SCHLEIF** ; **THOMAS VILLMANN** ; **MICHAEL BICHL**. Limited Rank Matrix Learning - Discriminative Dimension Reduction and Visualization. *Neural Networks*, February 2012, vol. 26 (4), 159-173
- **BARBARA HAMMER** ; **MARC STRICKERT** ; **THOMAS VILLMANN**. On the generalization ability of grlvq networks. *Neural Processing Letters*, 2005, vol. 21 (2), 109-120
- **BARBARA HAMMER** ; **THOMAS VILLMANN**. Generalized relevance learning vector quantization. *Neural Networks*, 2002, vol. 15 (8-9), 1059-1068
- **A.S. SATO** ; **K. YAMADA**. Generalized learning vector quantization. *Advances in Neural Information Processing Systems*, 1996, vol. 8, 423-429
- Relevance matrices in learning vector quantization. **P, SCHNEIDER** ; **M, MICHL** ; **B. HAMMER.** Proc. of the 15th European Symposium on Artificial Neural Networks (ESANN). D-side publishing, 2007, 37-43
- **NITESH V. et al.** *J. Artificial Intelligence Research*, 2002, vol. 16, 321-357
- **P.T. FLETCHER et al.** *IEEE Trans. On Medical Imaging*, 2004, vol. 23 (8), 995-1005
- **R.C. WILSON et al.** *IEEE Trans. Pattern Anal. Mach. Intell*, 2014, vol. 36 (11), 2255-2269